# EUROPEAN PATENT SPECIFICATION

(11) **EP 3 435 880 B1**
(45) Date of publication and mention of the grant of the patent: **19.08.2020**
(21) Application number: 17714727.9
(22) Date of filing: 30.03.2017
(51) Int. Cl.: A61B 8/12, A61B 8/00, B06B 1/06, H05K 1/00

(54) **FLEXIBLE SUPPORT MEMBER FOR INTRAVASCULAR IMAGING DEVICE AND ASSOCIATED DEVICES, SYSTEMS, AND METHODS**
FLEXIBLES TRÄGERELEMENT FÜR VORRICHTUNG ZUR INTRAVASKULÄREN BILDGEBUNG SOWIE ZUGEHÖRIGE VORRICHTUNGEN, SYSTEME UND VERFAHREN
ÉLÉMENT DE SUPPORT FLEXIBLE POUR DISPOSITIF D'IMAGERIE INTRAVASCULAIRE, ET DISPOSITIFS, SYSTÈMES ET PROCÉDÉS ASSOCIÉS

(30) Priority: 30.03.2016 US 201662315406 P
(43) Date of publication of application: 06.02.2019
(73) Proprietor: Koninklijke Philips N.V., 5656 AG Eindhoven (NL)
(72) Inventor: SAROHA, Princeton, 5656 AE Eindhoven (NL); MINAS, Maritess, 5656 AE Eindhoven (NL); STIGALL, Jeremy, 5656 AE Eindhoven (NL); WROLSTAD, David, Kenneth, 5656 AE Eindhoven (NL)
(74) Representative: Philips Intellectual Property & Standards
(86) International application number: PCT/EP2017/057554
(87) International publication number: WO 2017/167883

(56) References cited:
- WO-A2-03/088817
- US-A1- 2014 187 960

## Description

### TECHNICAL FIELD

The present disclosure relates generally to intravascular ultrasound (IVUS) imaging and, in particular, to the distal structure of an intravascular imaging device. For example, the distal structure can include a support structure and a flex circuit that are arranged to facilitate efficient assembly and operation of the intravascular imaging device.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state (also known as synthetic-aperture) IVUS catheters are one of the two types of IVUS devices commonly used today, the other type being the rotational IVUS catheter. Solid-state IVUS catheters carry a scanner assembly that includes an array of ultrasound transducers distributed around its circumference along with one or more integrated circuit controller chips mounted adjacent to the transducer array. The controllers select individual transducer elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

Manufacturing an intravascular imaging device that can efficiently traverse physiology within the human body is challenging. In that regard, components at the distal portion of the imaging device causes an area of high rigidity in the intravascular device, which increase the likelihood of kinking as the intravascular is steered through vasculature. A document US 2014/187960 A1 relates to solid-state intravascular ultrasound (IVUS) imaging devices, systems, and methods. One disclosed intravascular ultrasound (IVUS) device includes: a flexible elongate member; an ultrasound scanner assembly disposed at a distal portion of the flexible elongate member, the ultrasound scanner assembly including an ultrasound transducer array; an interface coupler disposed at a proximal portion of the flexible elongate member; and a cable disposed within and extending along a length of the flexible elongate member between the ultrasound scanner assembly and the interface coupler. Another document WO 03/088817 A2 relates to a method and apparatus for identifying and stabilizing vulnerable plaque via multifunctional catheters having both infrared detection and imaging capabilities.

Thus, there remains a need for an intravascular ultrasound imaging system that overcomes the limitations of a rigid imaging assembly while achieving efficient assembly and operation.

### SUMMARY

An intravascular ultrasound imaging system according to the invention is defined in independent claim 1. Further aspects are defined in dependent claims 2-10. A method of assembling an intravascular imaging device according to the invention is defined in independent claim 11. Further aspects are defined in dependent claims 12-15.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a diagrammatic schematic view of an imaging system, according to aspects of the present disclosure.
Fig. 2 is a diagrammatic top view of a scanner assembly in a flat configuration, according to aspects of the present disclosure.
Fig. 3 is a diagrammatic side view of a scanner assembly in a rolled configuration around a support member, according to aspects of the present disclosure.
Fig. 4 is a diagrammatic cross-sectional side view of a distal portion of an intravascular device, according to aspects of the present disclosure.
Fig. 5 is a diagrammatic top view of a scanner assembly in a flat configuration, according to aspects of the present disclosure.
Fig. 6 is a diagrammatic side view of a support member blank, according to aspects of the present disclosure.
Fig. 7A is a diagrammatic side view of a support member, according to aspects of the present disclosure.
Fig. 7B is a diagrammatic top view of the support member of Fig. 8A, according to aspects of the present disclosure.
Fig. 8A is a diagrammatic side view of a scanner assembly in a rolled configuration around the support member of FIG. 7A, according to aspects of the present disclosure.
Fig. 8B is a diagrammatic top view of a scanner assembly in a rolled configuration around a support member of FIG. 7A, according to aspects of the present disclosure.
Fig. 9 is a diagrammatic cross-sectional side view of the scanner assembly of FIG. 9A.
Fig. 10 is a diagrammatic side view of a scanner assembly in a rolled configuration around a support member, according to aspects of the present disclosure.
Fig. 11 is a diagrammatic side view of a support member, according to aspects of the present disclosure.
Fig. 12 is a diagrammatic side view of a support member, according to aspects of the present disclosure.
Fig. 13 is a flow diagram of a method of assembling an intravascular device, according to aspects of the present disclosure.
Figs. 14A and 14B are a diagrammatic perspective view of a flex circuit, according to aspects of the present disclosure.
Fig. 15 is a diagrammatic schematic view of an imaging assembly, including spaced support member components, according to aspects of the present disclosure.
Fig. 16 is a diagrammatic schematic view of an imaging assembly, including a distal portion of a flex circuit positioned around a support member, according to aspects of the present disclosure.
Figs. 17A, 17B, and 17C are diagrammatic perspective views of an intravascular device, according to aspects of the present disclosure.
Fig. 18 is a diagrammatic perspective view of an intravascular device, including an imaging assembly with a flex circuit and a distal support member, according to aspects of the present disclosure.
Fig. 19 is a diagrammatic perspective view of the intravascular device of Fig. 18 further including an inner proximal member.
Fig. 20 is a diagrammatic perspective view of the intravascular device of Fig. 19 further including an outer proximal member.

*In this specifications the following non-SI unit is used, which may be converted to the respective SI unit according to the following conversion table:*

| *Name of unit* | *Symbol* | *Conversion factor* | *SI or metric unit* |
|---|---|---|---|
| *Inch* | " | 2,54 | *cm* |

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the focusing system is described in terms of cardiovascular imaging, it is understood that it is not intended to be limited to this application. The system is equally well suited to any application requiring imaging within a confined cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

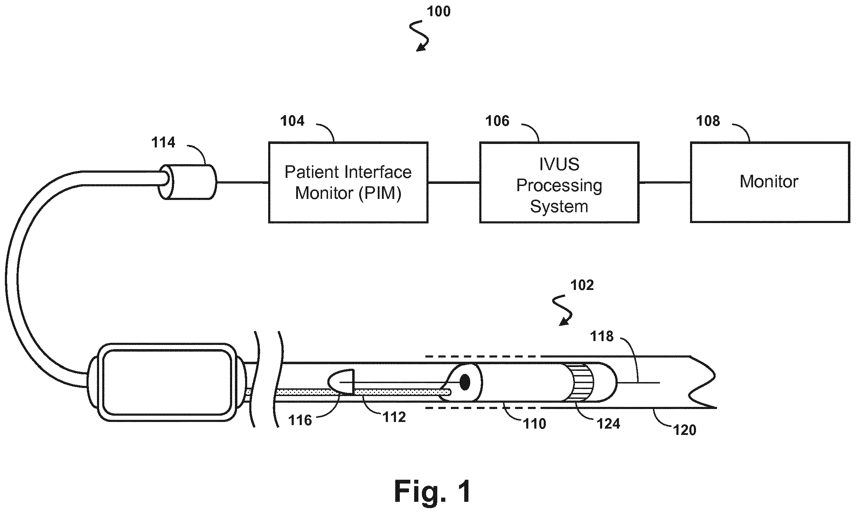

Fig. 1 is a diagrammatic schematic view of an intravascular ultrasound (IVUS) imaging system 100, according to aspects of the present disclosure. The IVUS imaging system 100 may include a solid-state IVUS device 102 such as a catheter, guide wire, or guide catheter, a patient interface module (PIM) 104, an IVUS processing system or console 106, and a monitor 108.

At a high level, the IVUS device 102 emits ultrasonic energy from a transducer array 124 included in scanner assembly 110 mounted near a distal end of the catheter device. The ultrasonic energy is reflected by tissue structures in the medium, such as a vessel 120, surrounding the scanner assembly 110, and the ultrasound echo signals are received by the transducer array 124. The PIM 104 transfers the received echo signals to the console or computer 106 where the ultrasound image (including the flow information) is reconstructed and displayed on the monitor 108. The console or computer 106 can include a processor and a memory. The computer or computing device 106 can be operable to facilitate the features of the IVUS imaging system 100 described herein. For example, the processor can execute computer readable instructions stored on the non-transitory tangible computer readable medium.

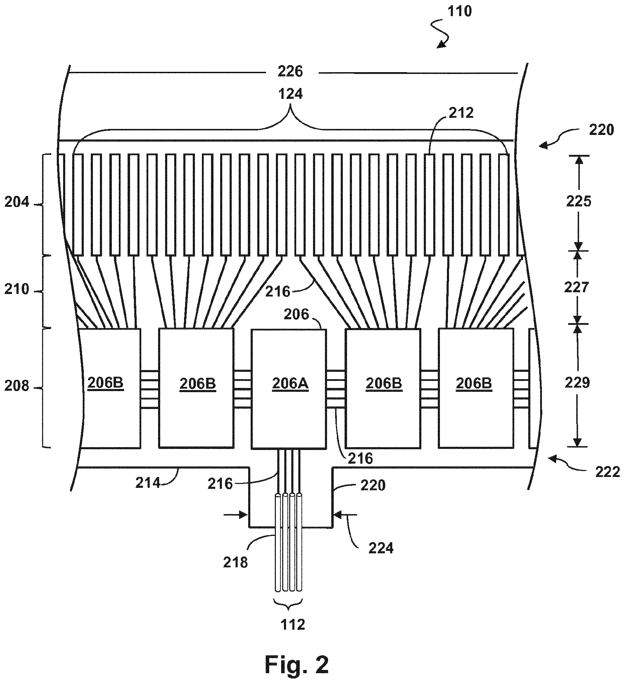

The PIM 104 facilitates communication of signals between the IVUS console 106 and the scanner assembly 110 included in the IVUS device 102. This communication includes the steps of: (1) providing commands to integrated circuit controller chip(s) 206A, 206B, illustrated in Fig. 2, included in the scanner assembly 110 to select the particular transducer array element(s) to be used for transmit and receive, (2) providing the transmit trigger signals to the integrated circuit controller chip(s) 206A, 206B included in the scanner assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s) 126 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The IVUS console 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be may be used to examine man-made structures such as, but without limitation, heart valves, stents, shunts, filters and other devices.

In some embodiments, the IVUS device includes some features similar to traditional solid-state IVUS catheters, such as the EagleEye® catheter available from Volcano Corporation and those disclosed in U.S. Patent No. 7,846,101. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). It is understood that any suitable gauge wire can be used for the conductors 218. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

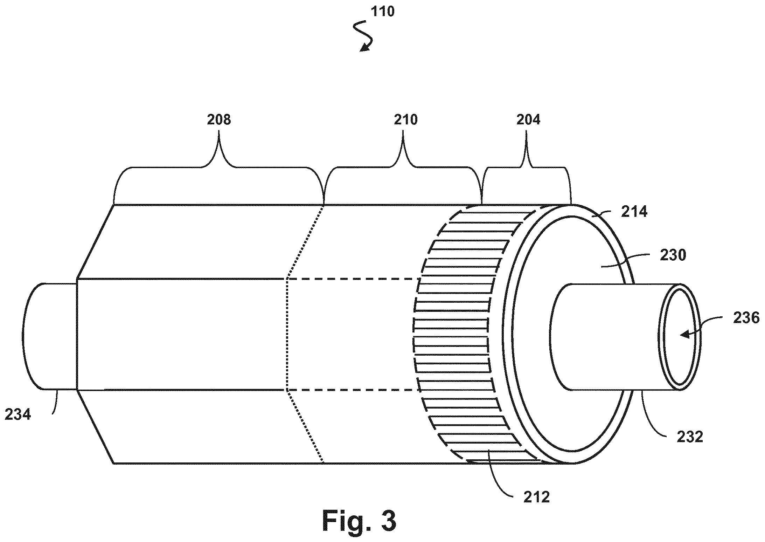

Fig. 2 is a top view of a portion of an ultrasound scanner assembly 110 according to an embodiment of the present disclosure. The assembly 110 includes a transducer array 124 formed in a transducer region 204 and transducer control logic dies 206 (including dies 206A and 206B) formed in a control region 208, with a transition region 210 disposed therebetween. The transducer control logic dies 206 and the transducers 212 are mounted on a flex circuit 214 that is shown in a flat configuration in Fig. 2. Fig. 3 illustrates a rolled configuration of the flex circuit 214. The transducer array 202 is a non-limiting example of a medical sensor element and/or a medical sensor element array. The transducer control logic dies 206 is a non-limiting example of a control circuit. The transducer region 204 is disposed adjacent a distal portion 220 of the flex circuit 214. The control region 208 is disposed adjacent the proximal portion 222 of the flex circuit 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or a length 227 of the transition region 210 can be greater than lengths 225, 229 of the transducer region and controller region, respectively. While the imaging assembly 110 is described as including a flex circuit, it is understood that the transducers and/or controllers may be arranged to form the imaging assembly 110 in other configurations, including those omitting a flex circuit.

The transducer array 124 may include any number and type of ultrasound transducers 212, although for clarity only a limited number of ultrasound transducers are illustrated in Fig. 2. In an embodiment, the transducer array 124 includes 64 individual ultrasound transducers 212. In a further embodiment, the transducer array 124 includes 32 ultrasound transducers 212. Other numbers are both contemplated and provided for. With respect to the types of transducers, in an embodiment, the ultrasound transducers 124 are piezoelectric micromachined ultrasound transducers (PMUTs) fabricated on a microelectromechanical system (MEMS) substrate using a polymer piezoelectric material, for example as disclosed in U.S. Patent 6,641,540. In alternate embodiments, the transducer array includes piezoelectric zirconate transducers (PZT) transducers such as bulk PZT transducers, capacitive micromachined ultrasound transducers (cMUTs), single crystal piezoelectric materials, other suitable ultrasound transmitters and receivers, and/or combinations thereof.

The scanner assembly 110 may include various transducer control logic, which in the illustrated embodiment is divided into discrete control logic dies 206. In various examples, the control logic of the scanner assembly 110 performs: decoding control signals sent by the PIM 104 across the cable 112, driving one or more transducers 212 to emit an ultrasonic signal, selecting one or more transducers 212 to receive a reflected echo of the ultrasonic signal, amplifying a signal representing the received echo, and/or transmitting the signal to the PIM across the cable 112. In the illustrated embodiment, a scanner assembly 110 having 64 ultrasound transducers 212 divides the control logic across nine control logic dies 206, of which five are shown in Fig. 2. Designs incorporating other numbers of control logic dies 206 including 8, 9, 16, 17 and more are utilized in other embodiments. In general, the control logic dies 206 are characterized by the number of transducers they are capable of driving, and exemplary control logic dies 206 drive 4, 8, and/or 16 transducers.

The control logic dies are not necessarily homogenous. In some embodiments, a single controller is designated a master control logic die 206A and contains the communication interface for the cable 112. Accordingly, the master control circuit may include control logic that decodes control signals received over the cable 112, transmits control responses over the cable 112, amplifies echo signals, and/or transmits the echo signals over the cable 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

The flex circuit 214, on which the transducer control logic dies 206 and the transducers 212 are mounted, provides structural support and interconnects for electrical coupling. The flex circuit 214 may be constructed to include a film layer of a flexible polyimide material such as KAPTON™ (trademark of DuPont). Other suitable materials include polyester films, polyimide films, polyethylene napthalate films, or polyetherimide films, other flexible printed semiconductor substrates as well as products such as Upilex® (registered trademark of Ube Industries) and TEFLON® (registered trademark of E.I. du Pont). In the flat configuration illustrated in Fig. 2, the flex circuit 214 has a generally rectangular shape. As shown and described herein, the flex circuit 214 is configured to be wrapped around a support member 230 (Fig. 3) to form a cylindrical toroid in some instances. Therefore, the thickness of the film layer of the flex circuit 214 is generally related to the degree of curvature in the final assembled scanner assembly 110. In some embodiments, the film layer is between 5 µm and 100 µm, with some particular embodiments being between 12.7 µm and 25.1 µm.

To electrically interconnect the control logic dies 206 and the transducers 212, in an embodiment, the flex circuit 214 further includes conductive traces 216 formed on the film layer that carry signals between the control logic dies 206 and the transducers 212. In particular, the conductive traces 216 providing communication between the control logic dies 206 and the transducers 212 extend along the flex circuit 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 112 when the conductors 218 of the cable 112 are mechanically and electrically coupled to the flex circuit 214. Suitable materials for the conductive traces 216 include copper, gold, aluminum, silver, tantalum, nickel, and tin, and may be deposited on the flex circuit 214 by processes such as sputtering, plating, and etching. In an embodiment, the flex circuit 214 includes a chromium adhesion layer. The width and thickness of the conductive traces 216 are selected to provide proper conductivity and resilience when the flex circuit 214 is rolled. In that regard, an exemplary range for the thickness of a conductive trace 216 and/or conductive pad is between 10-50 µm. For example, in an embodiment, 20 µm conductive traces 216 are separated by 20 µm of space. The width of a conductive trace 216 on the flex circuit 214 may be further determined by the width of the conductor 218 to be coupled to the trace/pad.

The flex circuit 214 can include a conductor interface 220 in some embodiments. The conductor interface 220 can be a location of the flex circuit 214 where the conductors 218 of the cable 114 are coupled to the flex circuit 214. For example, the bare conductors of the cable 114 are electrically coupled to the flex circuit 214 at the conductor interface 220. The conductor interface 220 can be tab extending from the main body of flex circuit 214. In that regard, the main body of the flex circuit 214 can refer collectively to the transducer region 204, controller region 208, and the transition region 210. In the illustrated embodiment, the conductor interface 220 extends from the proximal portion 222 of the flex circuit 214. In other embodiments, the conductor interface 220 is positioned at other parts of the flex circuit 214, such as the distal portion 220, or the flex circuit 214 omits the conductor interface 220. A value of a dimension of the tab or conductor interface 220, such as a width 224, can be less than the value of a dimension of the main body of the flex circuit 214, such as a width 226. In some embodiments, the substrate forming the conductor interface 220 is made of the same material(s) and/or is similarly flexible as the flex circuit 214. In other embodiments, the conductor interface 220 is made of different materials and/or is comparatively more rigid than the flex circuit 214. For example, the conductor interface 220 can be made of a plastic, thermoplastic, polymer, hard polymer, etc., including polyoxymethylene (e.g., DELRIN®), polyether ether ketone (PEEK), nylon, and/or other suitable materials. As described in greater detail herein, the support member 230, the flex circuit 214, the conductor interface 220 and/or the conductor(s) 218 can be variously configured to facilitate efficient manufacturing and operation of the scanner assembly 110.

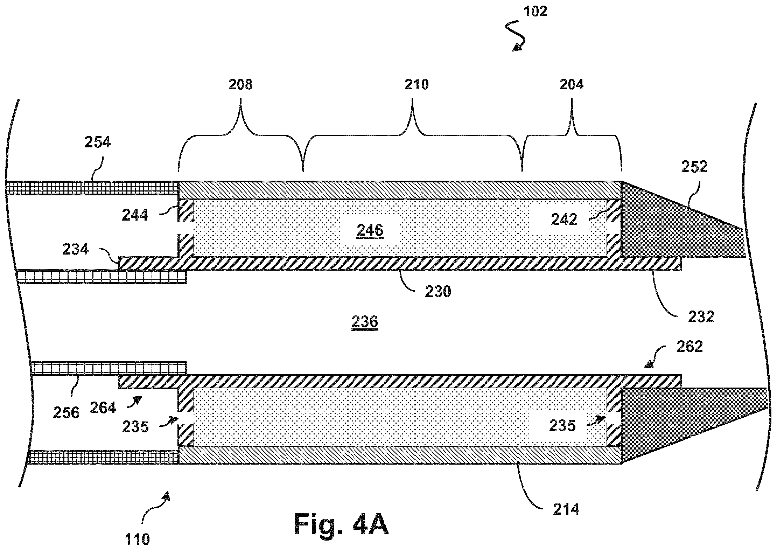

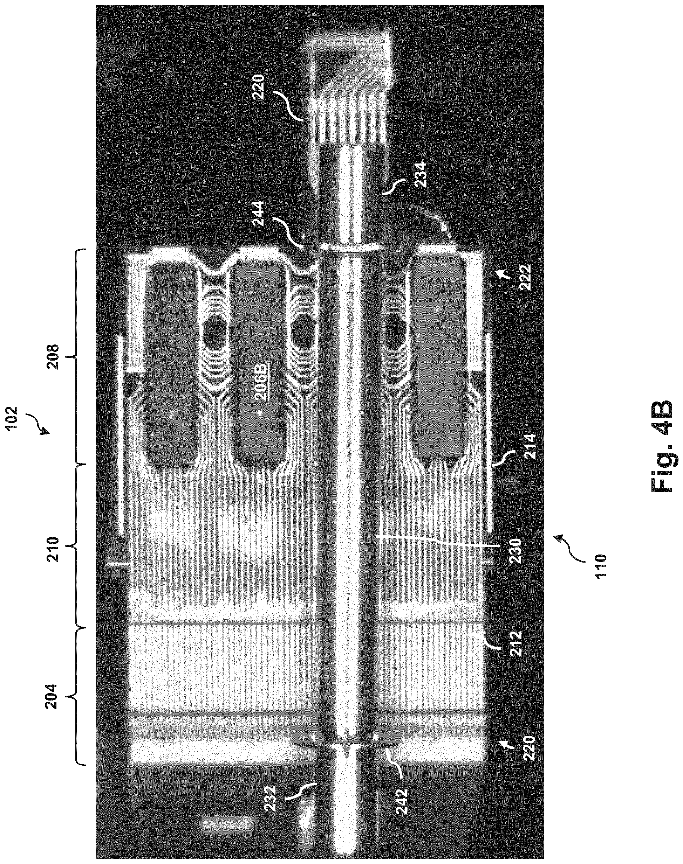

In some instances, the scanner assembly 110 is transitioned from a flat configuration (Fig. 2) to a rolled or more cylindrical configuration (Figs. 3 and 4). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND TRANSDUCER ASSEMBLY HAVING A FLEXIBLE SUBSTRATE".

As shown in Figs. 3 and 4, the flex circuit 214 is positioned around the support member 230 in the rolled configuration. Fig. 3 is a diagrammatic side view with the flex circuit 214 in the rolled configuration around the support member 230, according to aspects of the present disclosure. Fig. 4A is a diagrammatic cross-sectional side view of a distal portion of the intravascular device 110, including the flex circuit 214 and the support member 230, according to aspects of the present disclosure.

Fig. 4B illustrates the flex circuit 214 and the support member 230 prior to the flex circuit 214 being rolled around the support member 230.

The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014. -The support member 230 can be ferrule having a distal portion 262 and a proximal portion 264. The support member 230 can define a lumen 236 extending longitudinally therethrough. The lumen 236 is in communication with the exit port 116 and is sized and shaped to receive the guide wire 118 (Fig. 1). The support member 230 can be manufactured accordingly to any suitable process. For example, the support member 230 can be machined, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another.

Stands 242, 244 that extend vertically are provided at the distal and proximal portions 262, 264, respectively, of the support member 230. The stands 242, 244 elevate and support the distal and proximal portions of the flex circuit 214. In that regard, portions of the flex circuit 214, such as the transducer portion 204, can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244. To improve acoustic performance, any cavities between the flex circuit 214 and the surface of the support member 230 are filled with a backing material 246. The liquid backing material 246 can be introduced between the flex circuit 214 and the support member 230 via passageways 235 in the stands 242, 244. In some embodiments, suction can be applied via the passageways 235 of one of the stands 242, 244, while the liquid backing material 246 is fed between the flex circuit 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flex circuit 214. Fig. 4B may illustrate the flex circuit 214 being aligned with the support member 230 such that the distal portion 220 is positioned to be wrapped around the distal stand 242 and the proximal portion 222 is wrapped around the proximal stand 244.

The support member 230 can be substantially cylindrical in some embodiments. Other shapes of the support member 230 are also contemplated including geometrical, non-geometrical, symmetrical, non-symmetrical, cross-sectional profiles. Different portions the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can be flexible elongate member that extend from proximal portion of the intravascular 102, such as the proximal connector 114, to the imaging assembly 110. For example, the proximal inner member 256 can be received within a proximal flange 234. The proximal outer member 254 abuts and is in contact with the flex circuit 214. A distal member 252 is coupled to the distal portion 262 of the support member 230. The distal member 252 can be a flexible component that defines a distal most portion of the intravascular device 102. For example, the distal member 252 is positioned around the distal flange 232. The distal member 252 can abut and be in contact with the flex circuit 214 and the stand 242. The distal member 252 can be the distal-most component of the intravascular device 102.

One or more adhesives can be disposed between various components at the distal portion of the intravascular device 102. For example, one or more of the flex circuit 214, the support member 230, the distal member 252, the proximal inner member 256, and/or the proximal outer member 254 can be coupled to one another via an adhesive.

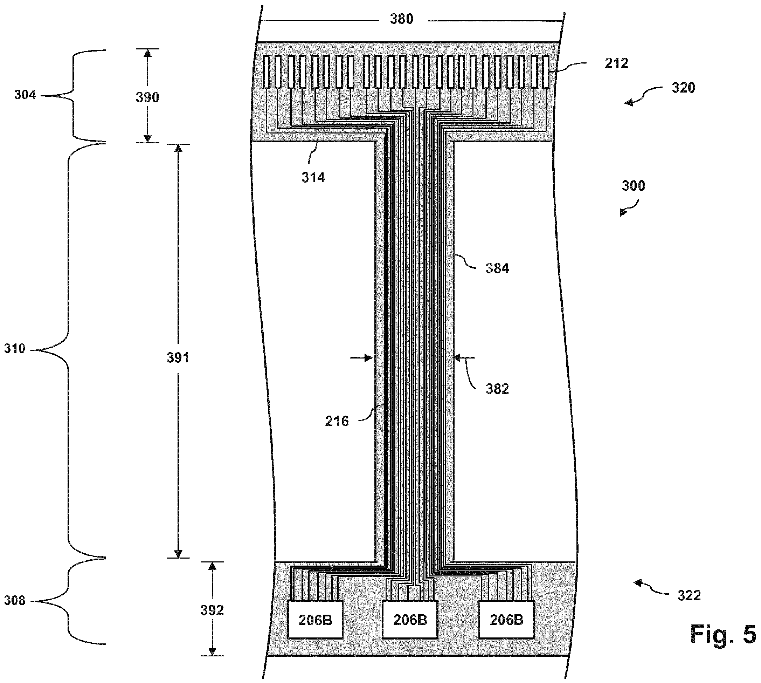

Fig. 5 illustrates exemplary embodiment of scanner assembly 300, including a flex circuit 314. At least a portion of the scanner assembly 300, such as a strip 384 of the transition region 310 can be shaped to improve flexibility of the intravascular device. As described herein, the support member around which the flex circuit 314 is positioned can be shaped to compliment the orientation of the strip 384 around the support member. The flex circuit 314 and the support member can be shaped to advantageously increase the flexibility of intravascular device and decrease the likelihood of kinking while the intravascular device is navigated through a patient's vasculature. The scanner assembly 300 can be positioned at a distal portion of an intravascular device. Fig. 5 is a diagrammatic top view of a scanner assembly 300 in a flat configuration.

The scanner assembly 300 and the flex circuit 314 can be similar in some respects to scanner assembly 110 and the flex circuit 214, respectively. The imaging assembly 300 includes a transducer region 304 having a plurality of transducers 212 at a distal portion 320 and a controller region 308 having plurality of controllers 206B at a proximal portion 322. A transition region 310 having a plurality of conductive traces 216 extending in a central portion between the distal and proximal portions 320, 322 facilitates communication between the plurality of transducers 212 and a plurality of controllers 206B. The transition region 310 comprises a strip 384 on which the conductive traces 216 are formed. One dimension of the strip 384, such as a width 382, can have a smaller value than a corresponding dimension, such as a width 380, of the transducer region 304 and/or the controller region 308. The width 382 of the transition region 310 and/or the strip 384 can be any suitable value, including between approximately 0.010" and 0.415". The width 380 of the transducer region 304 and/or the controller region 308 can be between approximately 0.081" and 0.415", for example. Another dimension of the strip 384, such as a length 391, can have a larger value than a corresponding dimension, such as lengths 390, 392 of the transducer region 304 and the controller region 308, respectively. The length 391 of the transition region 310 and/or the strip 384 can be any suitable value, including between approximately 0.005" and 0.500". The length 390 of the transducer region 304 can be between approximately 0.025" and 0.250", for example. The length 392 of the controller region 308 can be between approximately 0.025" and 0.250", for example.

Yet other orientations for the transition region 310 are contemplated . For example, the transition region 310 can include two or more strips. The two or more strips can be parallel or non-parallel. The one or more strips may intersect or overlap in some embodiments. One or more strips of the transition region 310 may extend at an oblique angle relative to the transducer and controller regions 304, 308.

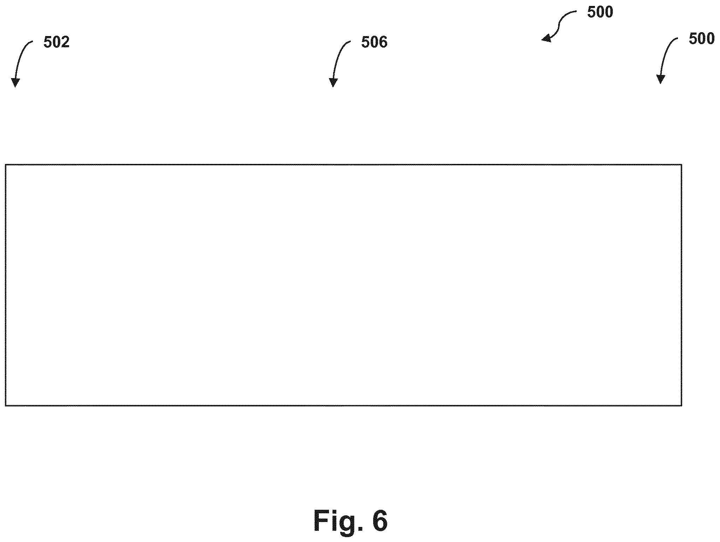

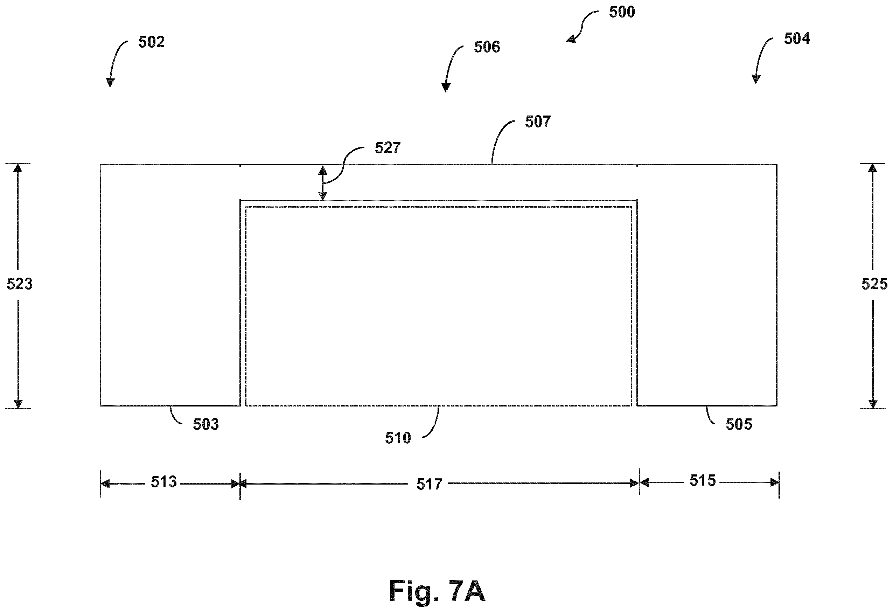

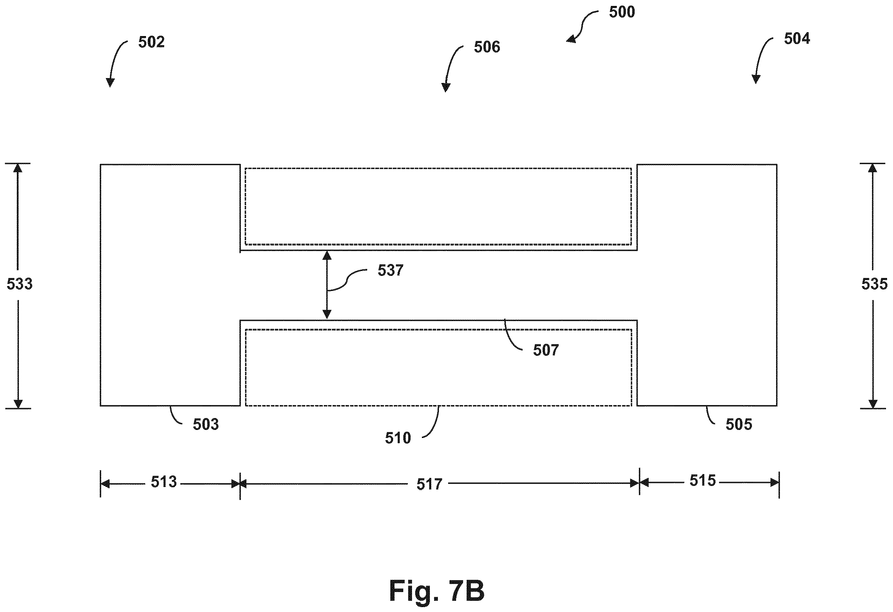

Figs. 6, 7A, and 7B illustrate stages of manufacturing an embodiment of a support member 500. The support member 500 may be sized and shaped to allow greater flexibility for the intravascular device. For example, the support member 500 may compliment the size and shape the flex circuit 314. Figs. 6 and 7A are diagrammatic side-views, and Fig. 7B is a diagrammatic top view.

Fig. 6 illustrates a side view of a blank of the support member 500. As described with respect to Figs. 7A and 7B, material may be removed from the blank to produce the support member 500 utilized in the intravascular device. As similarly described with respect to the support member 230, the support member 500 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014. The blank has a proximal portion 502, a distal portion 504, and a central portion 506 extending between the distal and proximal portions 502, 504. The blank may be variously sized and shaped in different embodiment. For example, while the illustrated embodiment shows a substantially cylindrical blank, the blank other shapes, such as a rectangular prism, ellipsoid, and/or other suitable shape.

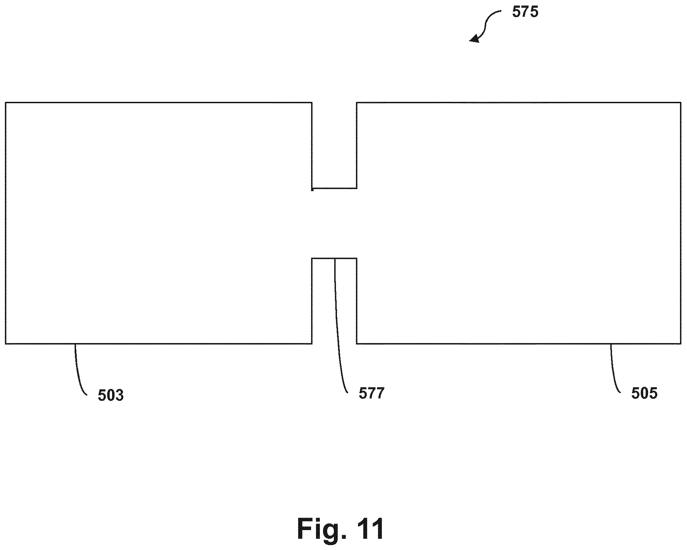

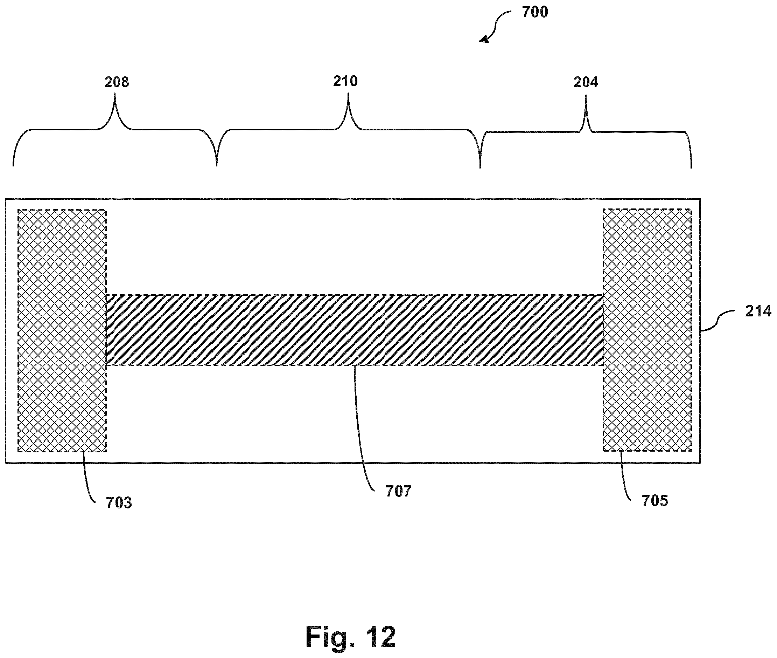

Figs. 7A and 7B show the support member 500 with a central section 507 formed between a proximal section 503 and a distal section 505. The support member 500 can be similar to the support members described herein, including the support member 230, in some aspects. The proximal and distal sections 503 can have an outer profile that is substantially cylindrically-shaped. The central section 507 can be a bridge extending between the proximal and distal sections 503, 505. In the illustrated embodiment, the central section 507 can be shaped a rectangular prism and extend in a linear manner between the proximal and distal sections 503, 505. For example, outer surfaces of the proximal, distal, and central sections 503, 505, 507 may form a continuous surface. In other embodiments, the central section may extend between the proximal and distal sections 503, 505 such that outer surface of the support member 500 is discontinuous (as shown in Figs. 11 and 12). Other configurations of the central section 507 are also contemplated. The support member 500 other features in different embodiments, such as a proximal and/or distal flange, which facilitates coupling to a flexible elongate proximal member and/or a distal member that defines a distal-most portion of the intravascular device.

One or more dimensions of the central section 507, such as a height 527 (Fig. 7A) and a width 537 (Fig. 7B), can have a smaller value than corresponding dimensions of the proximal and distal sections 503, 505. The height 527 of the central section 507 can be any suitable value, including between approximately 0.005" and 0.131". The width 537 of the central section 507 can be any suitable value, including between approximately 0.010" and 0.415". In some instances, the height 527 and/or width 537 can be based on cut, unrolled, and/or flattened state of the flex circuit 314. For example, the height 527 and/or width 537 of the support member 500 can match the dimensions (e.g., the width 382 and/or length 391) of the transition region 310 or the strip 384 of the flex circuit 314. Corresponding dimensions between the support structure 500 and the flex circuit 314 advantageously allows use of the support member 500 have a flexible bridge or central section 507. In some embodiments, the support member 500 includes shallow slots to create flex points within the support member structure.

The proximal and distal sections 503, 505 of the support member 500 can be variously sized and shaped to support the flex circuit when the flex circuit is positioned around the support member. The height 523 (Fig. 7A) of the proximal section 503 can be any suitable value, including between approximately 0.026" and 0.131". The width 533 (Fig. 7B) of the proximal section 503 can be any suitable value, including between approximately 0.026" and 0.131". The dimensions of the support member 500 can be selected such that the intravascular device 102 has a diameter between approximately 2 Fr and approximately 10 Fr, for example. In some instances, the height 523 and width 533 of the proximal section 503 can be equal, such as when the outer profile of the proximal section 503 is cylindrical.

In some embodiments, the proximal and distal sections 503, 505 of the support member 500 are similarly sized and shaped. In other embodiments, the proximal section 503 can be larger or smaller than the distal section 505. When the dimensions of the proximal and distal sections 503, 505 are different, the height 525 (Fig. 7A) of the distal section 505 can be any suitable value, including between approximately 0.026" and 0.131". The width 535 (Fig. 7B) of the distal section 505 can be any suitable value, including between approximately 0.026" and 0.131".

A length 513, 515, 517 of the proximal, distal, and central sections 503, 505, 507 can vary in different embodiments. In some instances, the length 517 of the central section 507 is larger than the lengths 513, 515 of the proximal and distal sections 503, 505, respectively. For example, the length 517 of the central section 507 can be any suitable value, including between approximately 0.005" and 0.500". The length 513 of the proximal section 503 can be between approximately 0.025" and 0.250", for example. The length 515 of the distal section 505 can be between approximately 0.025" and 0.250", for example. The lengths 513, 515, 517 may be based on the dimensions of the flex circuit 314, such as the length 392 of the controller region 308, the length 391 of the transition region 310, and the length 390 of the transducer region 304.

The proximal, distal, and central sections 503, 505, 507 can be integrally formed. In some embodiments, the support member 500 can be shaped by removing portions of the support member blank illustrated in Fig. 6. For example, the blank can be machined using a tool to remove the portions 510 indicated with broken lines. Removing the cutouts 510 advantageously increases flexibility of the support member 500 by reducing the amount of material contributing to the stiffness of the support member 500. In the illustrated embodiment, the cutouts 510 can be removed from the central section 507. The cutouts 510 can extend radially inwards from an outer surface of the support member 50f0. In some embodiments, the support member 500 is molded, such as with an injection molding process. In such instances, the support member 500 including the proximal, distal, and central sections 503, 505, 507, sized and shaped as in Figs. 7A, 7B, are formed without necessary removing the material indicated by cutouts 510. The proximal, distal, and central sections 503, 505, 507 can be integrally formed.

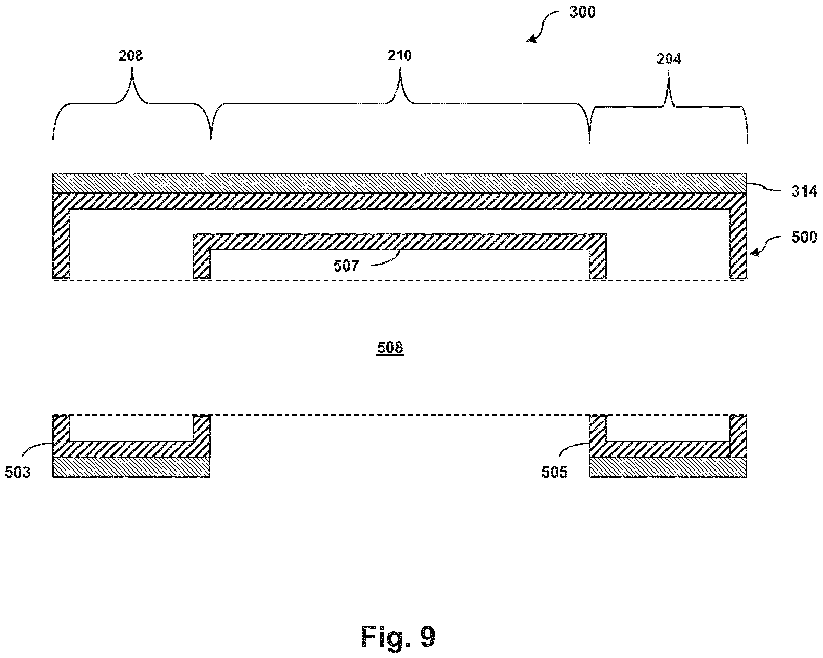

A lumen (e.g., the lumen 508 of Fig. 9) can extend longitudinally through the support member 500. In some embodiments, the blank (Fig. 6) of the support member 500 can include the lumen. In other embodiments, the lumen is formed by removing material extending through the longitudinal axis of the support member 500, such as when the material 510 is removed to shape the central section 507.

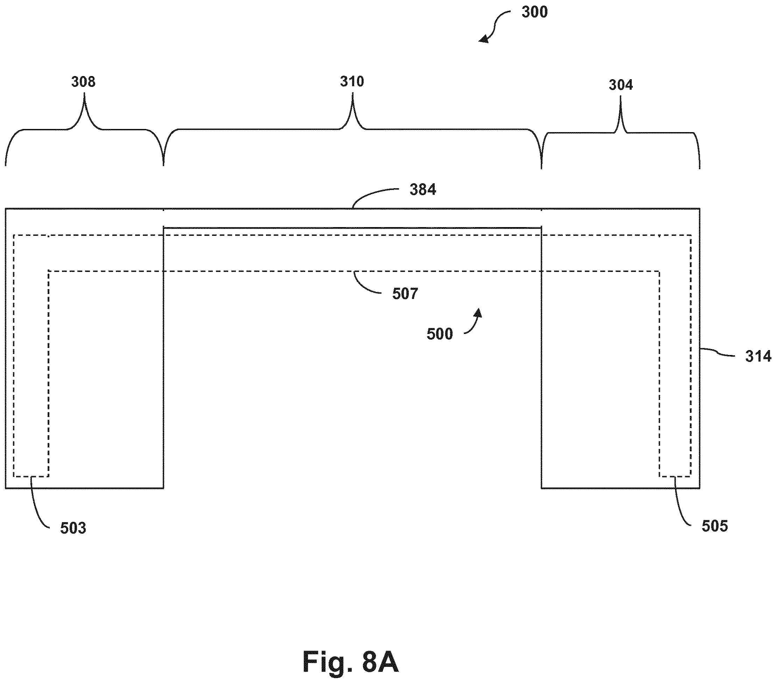

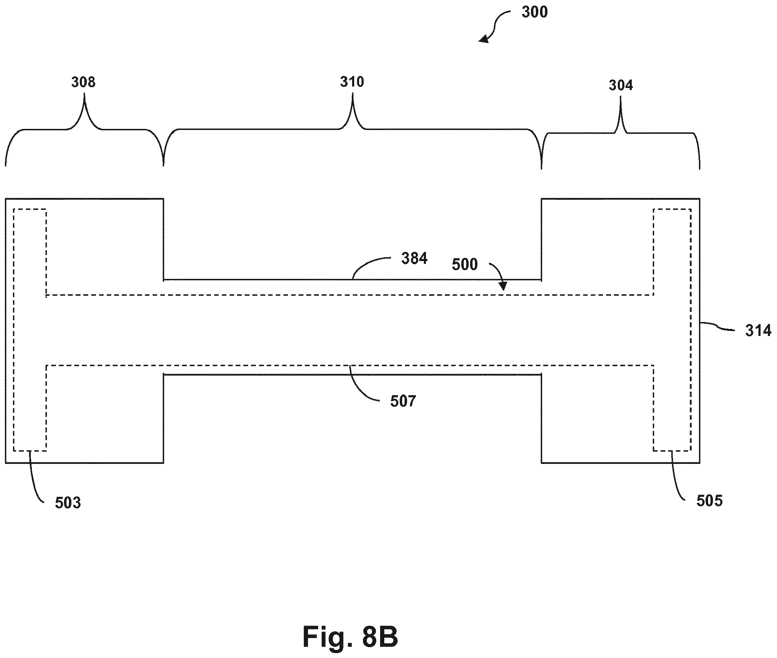

Figs. 8A, 8B, and 9 illustrate the imaging assembly 300, including the flex circuit 314 positioned around the support member 500. Fig. 8A is a diagrammatic side view of the imaging assembly 300, and FIG. 8B is a diagrammatic top view of the imaging assembly 300. Fig. 9 is a diagrammatic cross-sectional side view of the imaging assembly 300.

The proximal, distal, and central sections 503, 505, 507 of the support member 500 support the flex circuit 314 while advantageously minimizing the amount of material of the support member 500 and increasing flexibility of the imaging assembly 300. The transducer region 304 and the controller region 308 can have a cylindrical or cylindrical toroid configuration when rolled around the support member 500. A lumen 508, sized and shaped to receive the guide wire 118, extends longitudinally through the support member 500. The transducer region 304 of the flex circuit 314 is positioned around distal section 505 of the support member 500. The controller region 308 of the flex circuit 314 is positioned around the proximal section 503 of the support member 500.

The transition region 310 of the flex circuit 314, such as the strip 384, is aligned with the central portion 507 of the support member 500. The support member 500 can be shaped such that that central portion 507 supports the transition region 310. In that regard, the transition region 310 can be in contact with the central portion 507 while the flex circuit 314 is positioned around the support member 500.

Generally, a shape of the central portion 507 of the support member 500 corresponds to an orientation of at least a portion of the flex circuit 314, such as the transition region 310, around the support member. For example, as shown in Figs. 8A and 8B, the central portion 507 can extend between the proximal and distal portions 503, 505 in a linear manner when the transition region 310 between the transducer and controller regions 304, 308 extends in a linear manner.

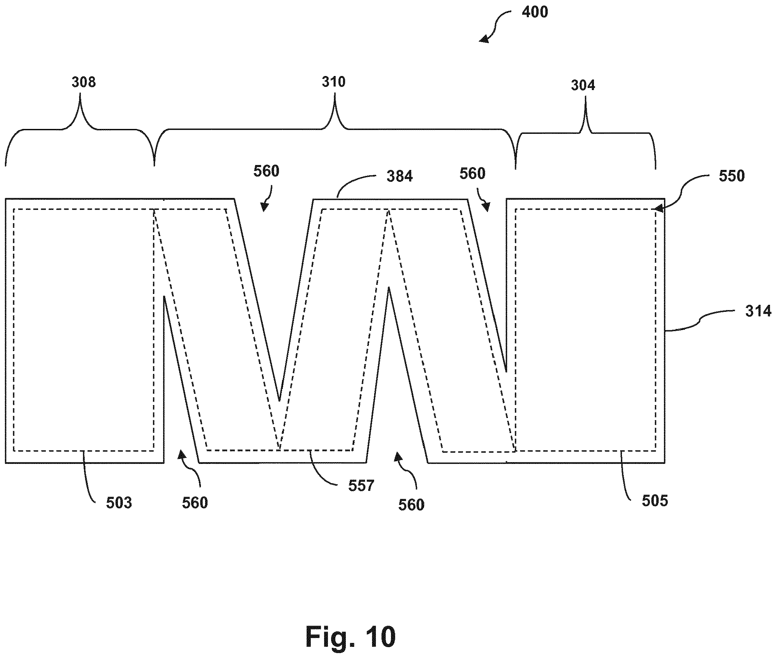

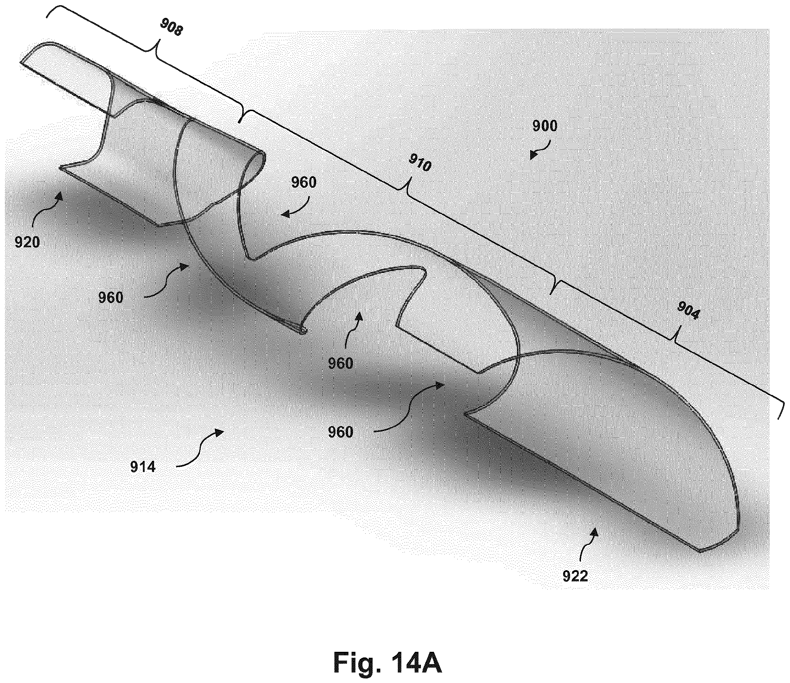

In other embodiments, the central portion 507 can extend between the proximal and distal portions 503, 505 in curved, spiral, and/or other suitable manner when the transition region 310 between the transducer and controller regions 304, 308 extends in a curved, spiral, and/or other suitable manner, respectively. For example, as shown in Fig. 10, the strip 384 of the transition region 310 is wrapped in a spiral configuration around the support member 550. Spiral wrapping of at least a portion of the flex circuit 314 is possible. The central section 557 of the support member 550 is shaped in a corresponding, spiral manner. In that regard, support member 550 includes multiple cutouts 560 such that the central section 557 acquires a spiral shape. The spiral windings of the strip 384 can be in contact with the spiral segments of the central portion 557. The spiral wrapping of the flex circuit 314 together with the spiral-shaped support member 550 may advantageously improve flexibility of the imaging assembly 400, such as while the intravascular device is traversing tortuous vessels within the body. Other exemplary shapes of the flex circuit 314 are illustrated in Figs. 14A, 14B, 17A, 17B, 17C, 18, 19, and 20.

The dimensions of the flex circuit 314 and/or the support member 500 may vary in different embodiment. For example, the proximal and/or distal portions may be sized to respectively accommodate controllers and transducers. In some instances, the flex circuit 314 may be space from the support member 500, and an acoustic backing material disposed between in a space between the flex circuit 314 and the support member 500 to facilitate operation of the transducers.

Fig. 11 illustrates a diagrammatic side view of a support member 575. The support member 700 can be similar to the support members described herein, including the support members 230, 500, 550, in some aspects. The support member 575 includes a flex joint 577 positioned between the proximal and distal portions 503, 505. The flex joint 577 advantageously enables the support member 575 to bend at the flex joint 577, which may increase maneuverability of the intravascular device within vasculature. The flex joint 577 may be described as a central portion of the support member 575 in some instances. The flex joint 577 may be a cylindrically-shaped connection member having a diameter that is smaller than the proximal and distal portion 503, 505. The flex joint 577 may be concentrically aligned with the proximal and distal portions 503, 505.

Fig. 12 illustrates a diagrammatic side of an imaging assembly 700, including a flex circuit 214 positioned around the support member 700. The support member 700 can be similar to the support members described herein, including the support members 230, 500, 550, 575, in some aspects. The support member 700 can be formed different material having differing degrees of flexibility. The support member 700 can also be formed of separate components that are fixedly joined. For example, the support member 700 can include a proximal portion 703, a distal portion 705, and a connection member 707. The support member 700 can be fixedly coupled to and extend between the proximal portion 703 and the distal portion 705. The flex joint 577 may be described as a central portion of the support member 575 in some instances. One or more of the proximal portion 703, the distal portion 705, and the connection member 707 can be formed of different materials. For example, the connection member 707 that is formed of a material that is more flexible than the materials of the proximal and distal portions 703, 705. In some instances, the connection member 707 may be formed of a nonmetallic material such a lower durometer Pebax, nylon, doped tungsten, copolymer/coextrusion, and/or a metallic material such as Nitinol, and/or other suitable material. In some instances, the proximal and distal portions 703, 705 can be formed of a nonmetallic material such as a higher durometer Pebax, nylon, doped tungsten, PEEK, polycarbonate, a metallic material such as stainless steel, and/or other suitable materials. While only one connection member 707 is shown, it is understood that two or more connection members can extend between the proximal and distal portions 703, 705 in some instances. Similarly, while the connection member 707 extends in a linear manner, other embodiments of the connection member can be arranged in a curved, spiral, and/or other suitable manner.

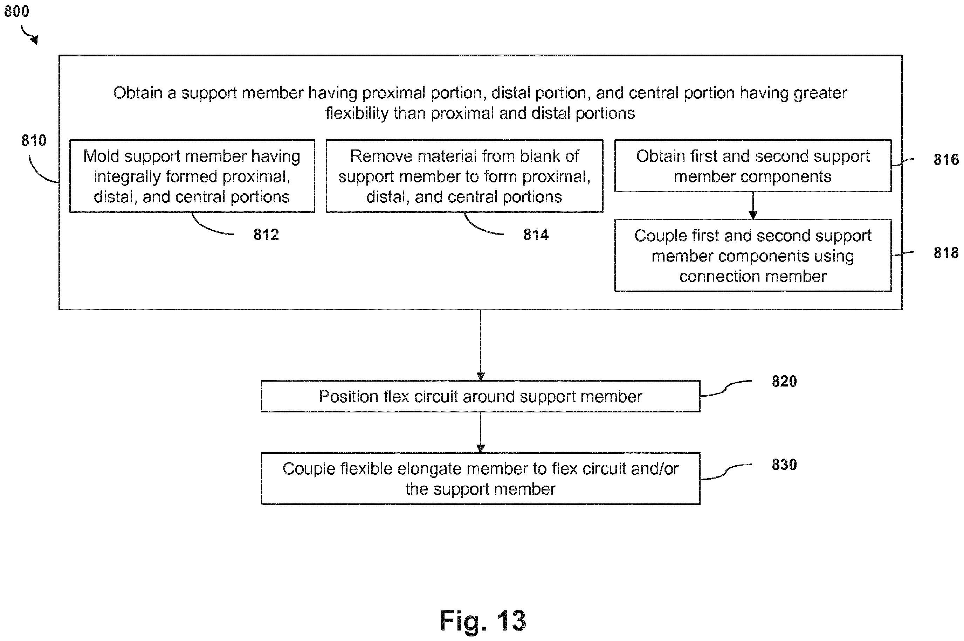

Fig. 13 is a flow diagram of a method 800 of assembling an intravascular imaging device, including an imaging assembly with a support member described herein. It is understood that the steps of method 800 may be performed in a different order than shown in Fig. 13, additional steps can be provided before, during, and after the steps, and/or some of the steps described can be replaced or eliminated in other embodiments. The steps of the method 800 can be carried out by a manufacturer of the intravascular imaging device.

At step 810, the method 800 includes obtaining a support member. The support member includes a proximal portion, a distal portion, and a central portion having greater flexibility than the proximal and distal portions. A value of a dimension of the central portion of the support member may less than values of the corresponding dimension of the proximal and distal portions. In some embodiment, obtaining the support member can include molding the support member (step 812), such as with injection molding and/or other suitable manufacturing process that integrally forms the proximal, distal, and central portions. In some embodiments, obtaining the support member can include machining the support member from a blank. For example, step 810 can include removing material from the black to form the proximal, distal, and central portions (step 814). In some instances, material can be removed from the blank to shape and/or form the central portion. In some embodiments, obtaining the support member can include joining separate and distinct components. For example, step 810 can include obtaining first and second support member components, such as proximal and distal components (step 816). Step 810 can additionally include coupling the first and second support member components using a connection member. One or more of the first and second support member components and the connection member can be formed of different materials in some instances. For example, the connection member may be formed of relatively more flexible material than the material of the first and second support member components.

At step 820, the method 800 includes positioning a flex circuit around a support member to form an imaging assembly of the intravascular device. The flex circuit can include a first section having a plurality of transducers, a second section having a plurality of controllers, and a third section having a plurality of conductive traces facilitating communication between the plurality of the transducers and the plurality of controllers. The flex circuit may initially be in a flat configuration. Step 820 can include transitioning at least a portion of the flex circuit into a rolled configuration around the support member. For example, the step 820 can include positioning the first, section, and/or third sections of the flex circuit around the support member. The step 820 can also include aligning the flex circuit with the support member, such as aligning the third section of the flex circuit having the conductive traces with the central portion of the support member. In some embodiments, the central portion of the support member can be shaped based on how the third section of the flex circuit is positioned around the support member, such as in a linear, curved, spiral, and/or other suitable manner.

At step 830, the method 800 can include coupling the flex circuit and/or the support member to one or more flexible elongate members. For example, one or more proximal flexible elongate members (e.g., an inner member and/or an outer member) are coupled to the flex circuit and/or the support member. In that regard, the flex circuit and/or the support member are positioned at the distal portion of the flexible elongate member. The method 800 can also include coupling the flex circuit and/or the support member to a distal component that defines a distal-most end of the intravascular imaging device. The method 800 can include introducing adhesive to affix the flex circuit and the support member and/or other components of the intravascular imaging device. The method 800 can also include introducing a liquid acoustic backing material between the flex circuit and the support member. A mandrel can be temporarily positioned around the support member to allow positioning of the flex circuit around the support member. The method 800 can also include removing the mandrel after the liquid acoustic backing material has cured.

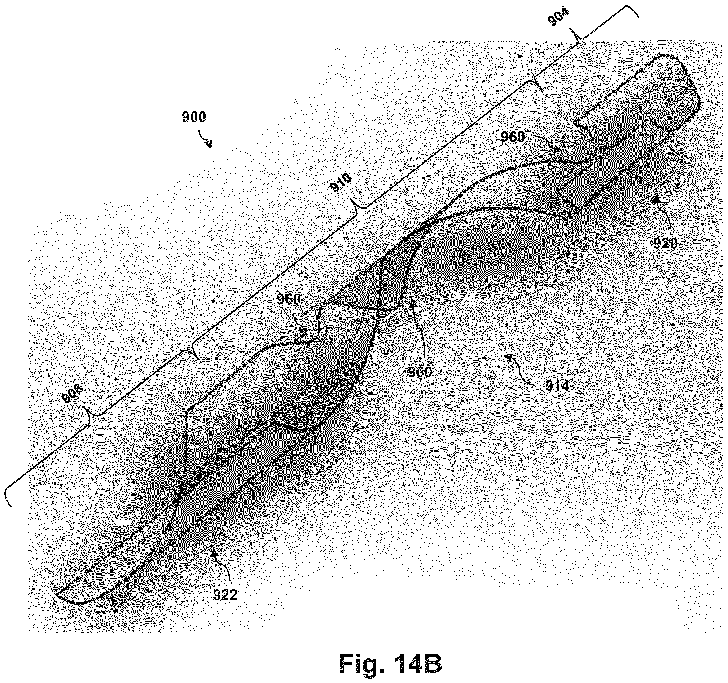

Figs. 14A and 14B illustrate an embodiment of an imaging assembly 900, including a flex circuit 914. The flex circuit 914 includes a controller region 904 at a proximal portion 922, a transducer region 908 at a distal portion 920, and a transition region 910 extending between the controller region 910 and the transducer region 908. The flex circuit 914 has a generally non-rectangular regular shape created by a plurality of cutouts 960. The cutouts 960 can be any suitable shape such that the shape of the flex circuit 914 is curved, linear, polygonal, ellipsoidal, and/or combinations thereof. For example, the cutouts 960 can be linear, rectangular, diamond, polygonal, ellipsoidal, curved, including C-curves, S-curves, Z-curves, etc., and/or combinations thereof. For example the flex circuit 914 may be generally spiral-shaped. In some embodiments, the cutouts 960 extend only within the transition region 910, while in other embodiments, the cutouts 960 may extend within any one or more of the proximal portion 922, the transducer region 908, and/or the transition region 910.

In some embodiments, one or more regions of the flex circuit 914 are wrapped in a curved or spiral manner around a support member. In other embodiments, the one or more regions of the flex circuit 914 are rolled around the support member as a rectangular-shaped flex circuit. That is, the cutouts 960 provide the non-rectangular, curved, and/or spiral shape to the flex circuit 914 even when flex circuit 914 is not itself wrapped in a curved or spiral manner.

The non-rectangular shape of the flex circuit 914 advantageously improves flexibility of the imaging assembly 900. The flex circuit 914 may have a continuous surface between the controller region 904 and the transducer region 908 allowing conductive traces to extend therebetween (e.g., within the transition region 910). One or more regions of the flex circuit 914 may be wrapped around a support member and/or directly around an outer, distal, flexible, and/or tubular member. For example, the transducer region 908 may be rolled around a support member, while the controller region 904 and/or transition region 910 are rolled directly around a distal member.

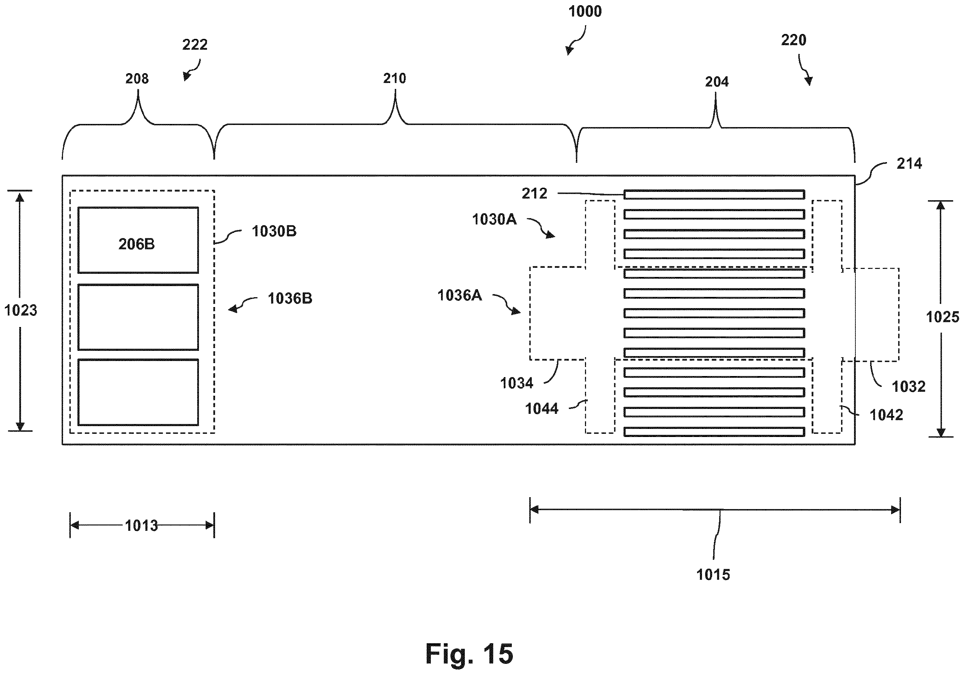

Fig. 15 illustrates an embodiment of an imaging assembly 1000, including the flex circuit 214 and two support member components 1030A, 1030B that are spaced from one another. The proximal portion 222 of the flex circuit 214, such as the controller region 208, is positioned around the support member 1030B. The distal portion 220 of the flex circuit 214, such as the transducer region 204, is positioned around the support member 1030A. The support member components 1030A, 1030B are positioned remote from one another along a length of the intravascular device. For example, the distal support member 1030A may be positioned relatively more distally along the length of the intravascular device than the proximal support member 1030B. The flex circuit 214, such as the transition region 210 extends between the support member components 1030A, 1030B. While a rectangular flex circuit 214, wrapped in cylindrical configuration, is shown and described with respect to Fig. 15, it is understood that any suitable flex circuit, including the flex circuits 314, 900 can be implemented in the imaging assembly 1000. For example, the flex circuit can have a non-rectangular shape, including cutouts as described with respect to the flex circuit 900.

The support member 1030A is similar to the support member 230 in some aspects. The support member 1030A can have a generally cylindrical shape with a substantially circular or ellipsoidal profile. The support member 1030A can have any other suitable shape in different embodiments, with a non-circular, polygonal cross-section. The support member 1030A may include a distal flange 1032, a proximal flange 1034. The support member 1030A can also include a distal stand 1042 and a proximal stand 1044. A central body portion of the support member 1030 extends between the distal and proximal stands 1042, 1044. The transducer region 204 of the flex circuit 214 is positioned around the support member 1030A in contact with the stands 1042, 1044 and radially spaced from the central body portion. The stand 1042 and /or stand 1044 can include one or more passageways to allow introduction of an acoustic backing material into the space between the flex circuit 214 and the central body portion of the support member 1030A. The support member 1030A can have a height or width 1025 between approximately 0.026" and 0.131", for example. A length 1015 of the support member can be between 0.005" and 0.500" or between approximately 0.025" and 0.250", for example.

The support member 1030B can be a generally cylindrically-shaped component that supports the proximal portion 222 of the flex circuit 214. In such embodiments, the cross-sectional of the support member 1030B may be circular or ellipsoidal. In other embodiments, the support member 1030B may be otherwise suitably shaped, including having a non-circular, polygonal cross-section. The controller region 208 can be in contact with an outer surface of the support member 1030B. The support member 1030B can have a height or width 1023 between approximately 0.026" and 0.131", for example. A length 1013 of the support member can be between 0.005" and 0.500" or between approximately 0.025" and 0.250", for example. The dimensions of the support members 1030A, 1030B can be selected such that the intravascular device has a diameter between approximately 2 Fr and approximately 10 Fr, for example.

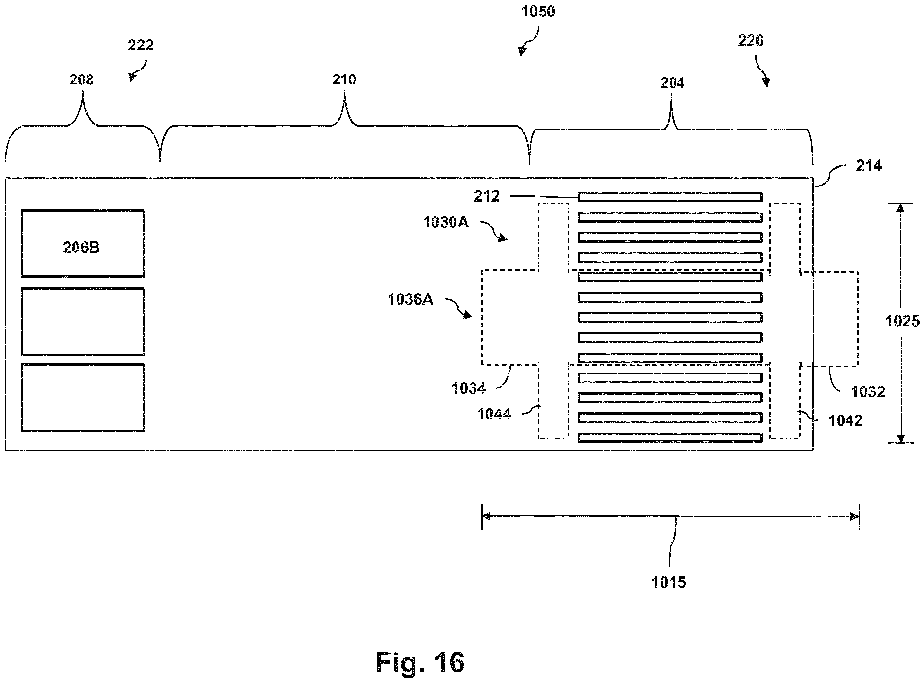

The support member 1030A, 1030B include lumens 1036A, 1036B, respectively. The lumens 1036A, 1036B can be sized and shaped to accommodate a flexible, inner, proximal member and/or a guide wire. In that regard, the inner proximal member can extend imaging assembly 1000, including through the lumens 1036A, 103B of support members 1030A, 1030B, and through the lumen created when the flex circuit 214 is in a rolled configuration. A flexible, outer, proximal member can be positioned around the controller region 208 and/or the transition region 210 of the flex circuit 214. While the transducer region 204 is positioned around the support member 1030A and the controller region 208 is positioned around the support member 1030B, the transition region 210 may be positioned directly around the inner proximal member. In some embodiments, as shown in Fig. 16, the proximal support member component (e.g., the support member 1030B around which the controller region 208 is positioned) is omitted. In such embodiments, the controller region 208 and the transition region 204 are positioned directly around the inner proximal member.

The arrangement of Figs. 15 and 16 advantageously improves the flexibility of the imaging assembly 1000 by reducing the length of the more rigid support member. Rather than a relatively long single support member, imaging assembly 1000 splits the support member into one or two relatively shorter support member segments 1030A, 1030B. Further, the flex circuit 214 extends between segments 1030A, 1030B which allows for bending in the imaging assembly 1000 within the transition region 210 of the flex circuit 214.

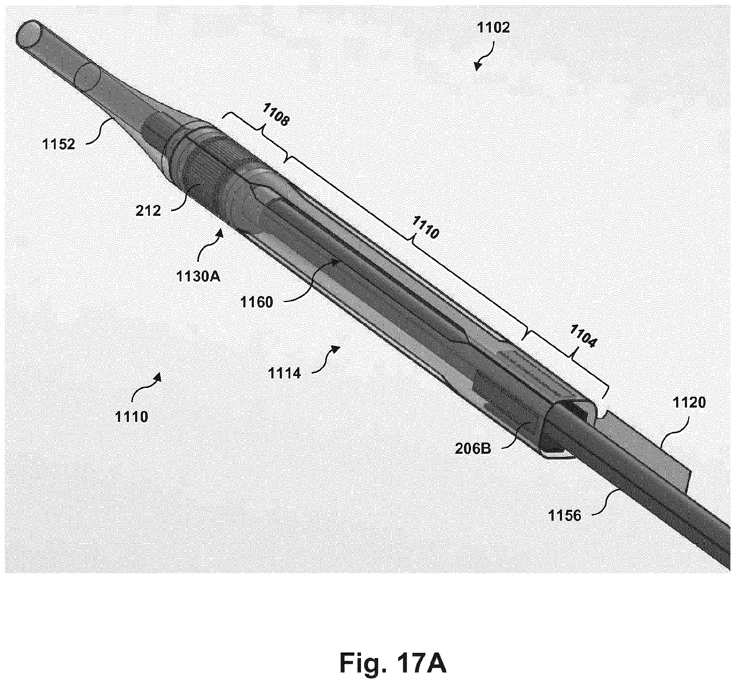

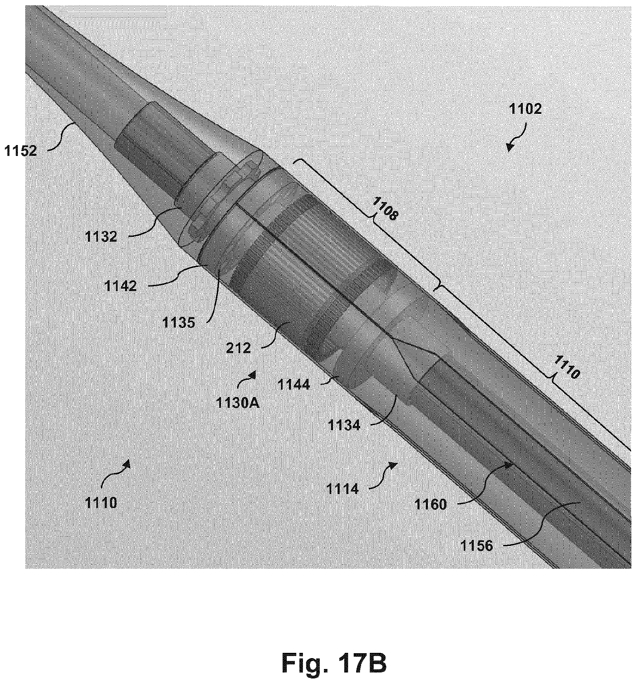

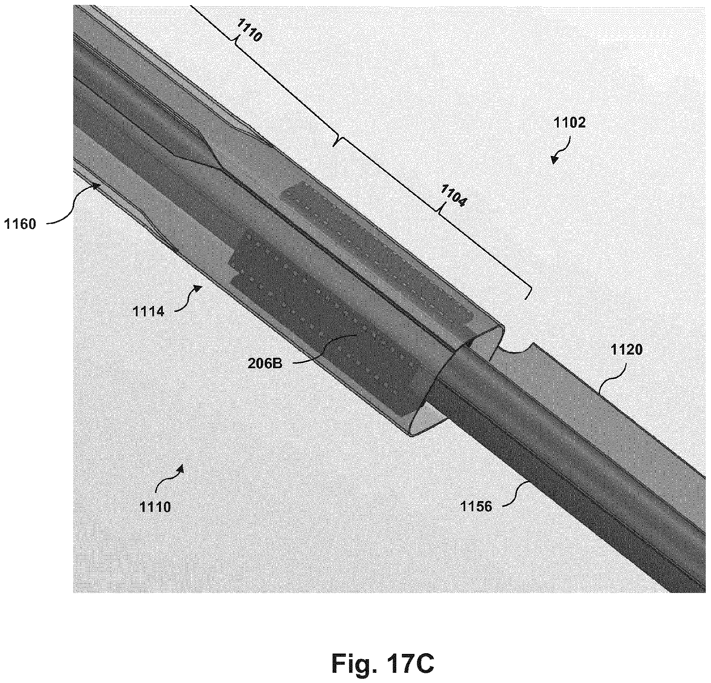

Figs. 17A, 17B, and 17C illustrate an embodiment of an intravascular device 1102. Fig. 17A illustrates a distal portion of the intravascular device 1102, including an imaging assembly 1110 having a flex circuit 1114 and the support member 1130A. Fig. 17B illustrates a distal portion of the imaging assembly 1110, and FIG. 17C illustrates a proximal portion of the imaging assembly 1110.

The flex circuit 1114 includes a controller region 1104 includes a plurality of controllers 206B, a transducer region 1108 includes a plurality of transducers 212, and a transition region 1110 extending between the controller region 1104 and the transducer region 1108. The transition region 1110 can include conductive traces facilitating electrical communication between the controllers 206B and the transducers 212. The flex circuit 1114 includes a conductor interface 1120 at which a plurality of electrical conductors are electrically coupled. The transition region 1110 includes a plurality of cutouts 1160, such as the diamond-shaped cutouts shown in the illustrated embodiment. The cutouts 1160 extend longitudinally within the transition region 1110. In that regard, the cutouts 1160 may be symmetrically disposed and/or disposed in a circumferential manner about a longitudinal axis of the intravascular device 1102.

The flex circuit 1114 is wrapped in a generally cylindrical or cylindrical toroid configuration. For example, the transducer region 1108 is wrapped around a distal support member 1130A. The flex circuit 1114 is in contact with a distal stand 1142 and a proximal stand 1144. The distal and proximal stands 1142, 1144 support and elevate the transducer region 1108 from a central body portion of the support member 1130A extending between the stands 1142, 1144. Acoustic backing material can be introduced into the radial space between the flex circuit 1114 and the central body portion of the support member 1130A via passageways 1135 of the distal stand 1142.

A longitudinal lumen extends through a distal flange 1132, the central body portion, and a proximal flange 1134 of the support member 1130A. A flexible, inner, proximal member 1156 extends through the longitudinal lumen. The proximal member 1156 may itself include a lumen sized and shaped to receive a guide wire. The transition region 1110 is wrapped directly around the proximal member 1156. In some instances, the intravascular device 1102 includes a flexible, outer proximal member that is positioned around the controller region 1104 and/or the transition region 1110.

A distal member 1152 defines a distal-most portion of the intravascular device 1102. The distal member 1152 extends from the distal stand 1142 and may be positioned around the distal flange 1132. The distal member 1152 may include a lumen in communication with a lumen of the inner member 1156 and sized and shaped to receive a guide wire. The distal member 1152 may abut and/or contact the flex circuit 1114 and/or the distal stand 1142.

Various components of the intravascular device 1102 may be coupled to one another via an adhesive. For example, the flex circuit 1114 may be coupled to the support member 1130A, such as the stands 1142, 1144, via an adhesive. The distal member 1152 may be coupled to the distal flange 1132, the stand 1142, and/or the flex circuit 1114 via an adhesive. The support member 1130A and/or the transition region 1110 may be coupled to the proximal member 1156 via an adhesive.

In some embodiments, the intravascular device includes a proximal support member. The controller region 1104 may be positioned around and in contact with the proximal support member. The proximal support member and the distal support member 1130A may be spaced from one another, with only the flex circuit 1114 extending therebetween. In some embodiments, the controller region 1104 is positioned directly around the proximal member 1156, without a proximal support member. For example, the controller region 1104 may be coupled to the proximal member 1156 via an adhesive. The non-rectangular shape of the transition region 1110, provided by the cutouts 1160, as well as the spaced distal support member 1130A and/or proximal support member, advantageously improve flexibility of the imaging assembly 1110 by providing relatively more bendable areas and reducing the amount of rigid materials.

In some embodiments, the cross-sectional profiles of controller region 1104, transducer region 1108, and/or transition region 1110 can be different. For example, the transducer region 1108 can have a generally circular or ellipsoidal cross-sectional profile. The controller region 1104 can have a polygonal cross-sectional profile. The transition region 1110 can have a circular, ellipsoidal, and/or polygonal cross-sectional profile.

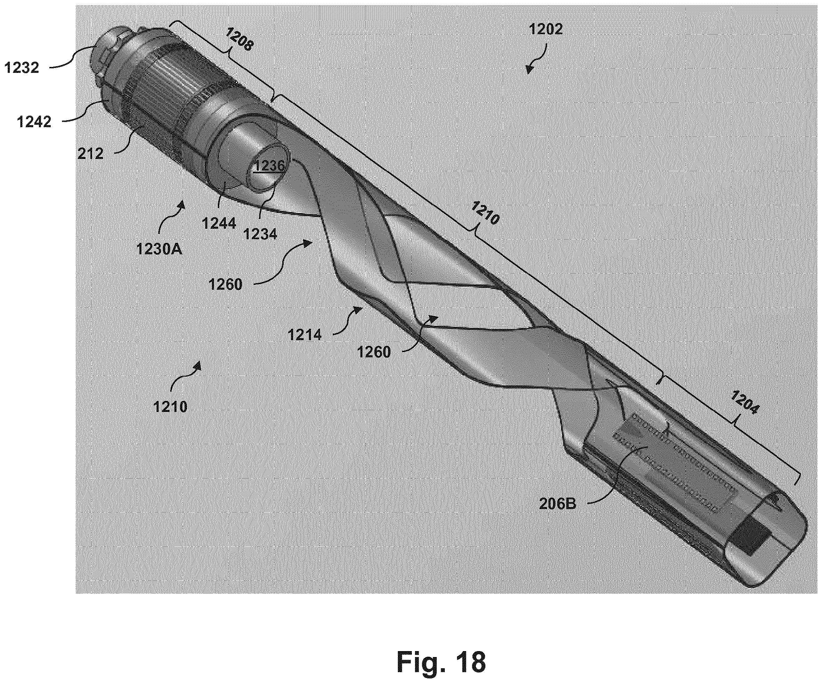

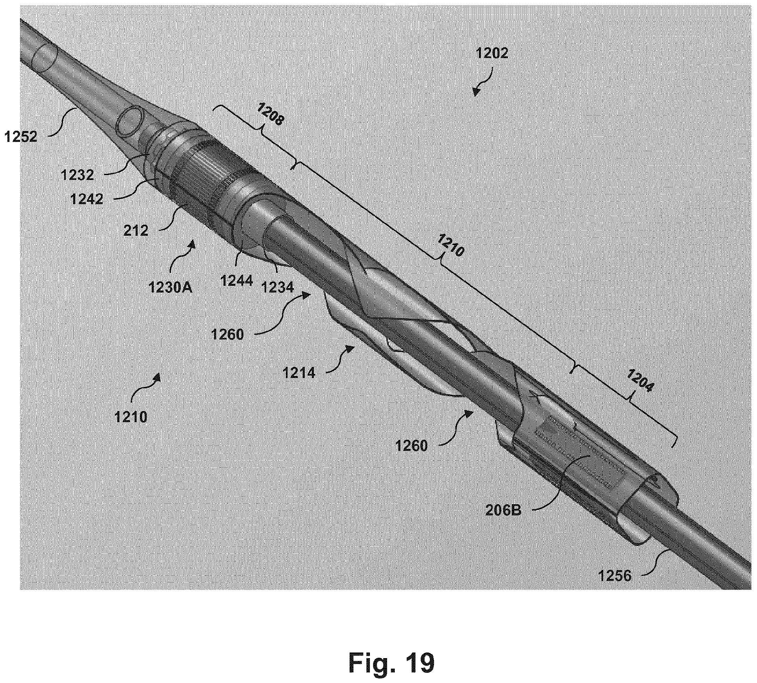

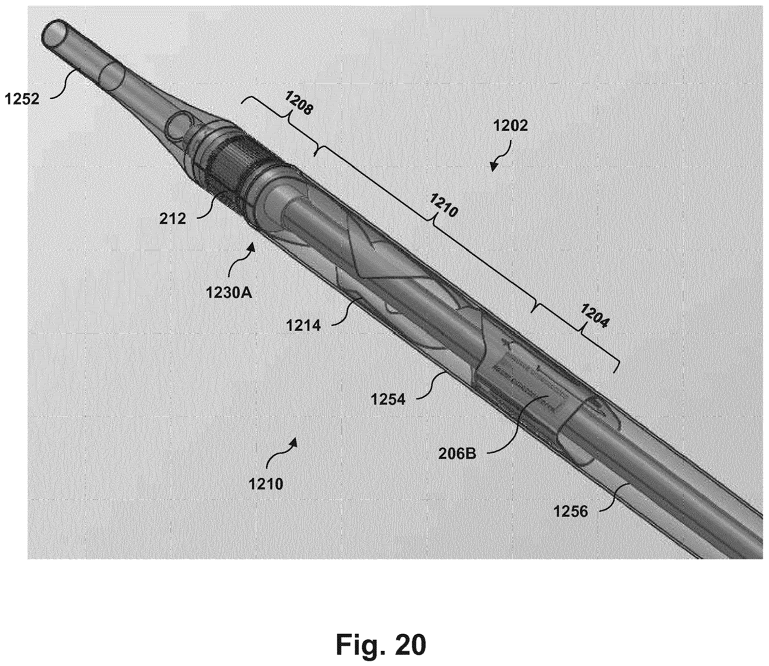

Figs. 18, 19, and 20 illustrate an embodiment of an intravascular device 1202. In that regard, Figs. 18, 19, 20 illustrate various combinations of elements of the intravascular device 1202. Fig. 18 illustrates an imaging assembly 1210, including a flex circuit 1214 and a distal support member 1230A. Fig. 19 illustrates the imaging assembly of Fig. 18 and additionally includes an inner proximal member 1256. Fig. 20 illustrates the imaging assembly of Fig. 19 and additionally includes an outer proximal member 1254.

The flex circuit 1214 includes a controller region 1204 includes a plurality of controllers 206B, a transducer region 1208 includes a plurality of transducers 212, and a transition region 1210 extending between the controller region 1204 and the transducer region 1208. The transition region 1210 can include conductive traces facilitating electrical communication between the controllers 206B and the transducers 212. The transition region 1210 includes a plurality of cutouts 1260, such as non-rectangular, curved, and/or spiral cutouts shown in the illustrated embodiment. The transition region 1210 includes two C-shaped strips that extending longitudinally and circumferentially between the controller region 1204 and the transducer region 1208.

The flex circuit 1214 is wrapped in a generally cylindrical or cylindrical toroid configuration. For example, the transducer region 1208 is wrapped around a distal support member 1230A. The flex circuit 1214 is in contact with a distal stand 1242 and a proximal stand 1244. The distal and proximal stands 1242, 1244 support and elevate the transducer region 1208 from a central body portion of the support member 1230A extending between the stands 1242, 1244. Acoustic backing material can be introduced into the radial space between the flex circuit 1214 and the central body portion of the support member 1230A via passageways of the distal stand 1242 and/or the proximal stand 1244.

A longitudinal lumen 1236 extends through a distal flange 1232, the central body portion, and a proximal flange 1234 of the support member 1230A. As shown in Fig. 19, the flexible, inner, proximal member 1256 extends through the longitudinal lumen. The proximal member 1256 may itself include a lumen sized and shaped to receive a guide wire. The transition region 1210 is wrapped directly around the proximal member 1256. As shown in Fig. 20, the intravascular device 1202 includes a flexible, outer proximal member that is positioned around the controller region 1204 and/or the transition region 1210.

A distal member 1252 defines a distal-most portion of the intravascular device 1202. The distal member 1252 extends from the distal stand 1242 and may be positioned around the distal flange 1232. The distal member 1252 may include a lumen in communication with a lumen of the inner member 1256 and sized and shaped to receive a guide wire. The distal member 1252 may abut and/or contact the flex circuit 1214 and/or the distal stand 1242.

Various components of the intravascular device 1202 may be coupled to one another via an adhesive. For example, the flex circuit 1214 may be coupled to the support member 1230A, such as the stands 1242, 1244, via an adhesive. The distal member 1252 may be coupled to the distal flange 1232, the stand 1242, and/or the flex circuit 1214 via an adhesive. The support member 1230A and/or the transition region 1210 may be coupled to the proximal member 1256 via an adhesive.

In some embodiments, the intravascular device includes a proximal support member. The controller region 1204 may be positioned around and in contact with the proximal support member. The proximal support member and the distal support member 1230A may be spaced from one another, with only the flex circuit 1214 extending therebetween. In some embodiments, the controller region 1204 is positioned directly around the proximal member 1256, without a proximal support member. For example, the controller region 1204 may be coupled to the proximal member 1256 via an adhesive. The non-rectangular shape of the transition region 1210, provided by the cutouts 1260, as well as the spaced distal support member 1230A and/or proximal support member, advantageously improve flexibility of the imaging assembly 1210 by providing relatively more bendable areas and reducing the amount of rigid materials.

In some embodiments, the cross-sectional profiles of controller region 1104, transducer region 1208, and/or transition region 1210 can be different. For example, the transducer region 1208 can have a generally circular or ellipsoidal cross-sectional profile. The controller region 1204 can have a polygonal cross-sectional profile. The transition region 1210 can have a circular, ellipsoidal, and/or polygonal cross-sectional profile.

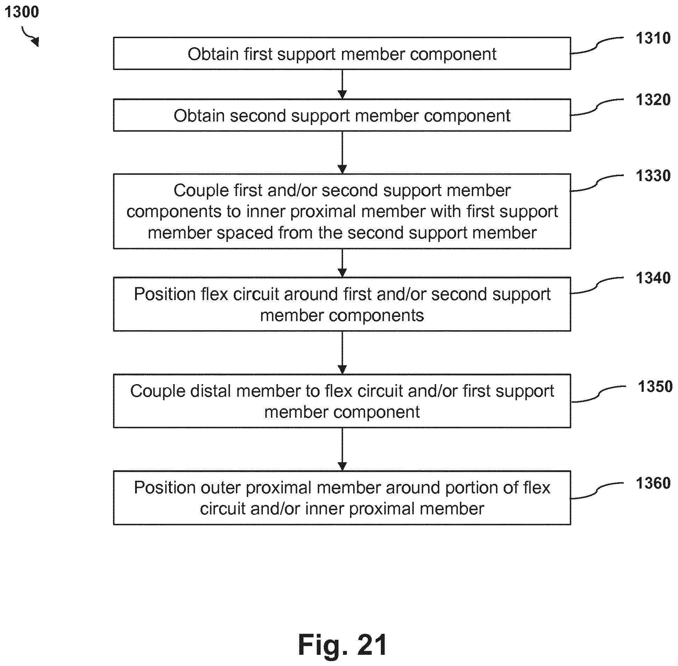

Fig. 21 is a flow diagram of a method 1300 of assembling an intravascular imaging device. It is understood that the steps of method 1300 may be performed in a different order than shown in Fig. 21, additional steps can be provided before, during, and after the steps, and/or some of the steps described can be replaced or eliminated in other embodiments. The steps of the method 1300 can be carried out by a manufacturer of the intravascular imaging device.

At step 1310, the method 1300 includes obtaining a first support member component. For example, the first support member component may be distal support member component. The first support member component may be sized and shaped so that a transducer region of a flex circuit can positioned around it.

At step 1320, the method 1330 includes obtaining a second support member component. For example, the second support member component may be proximal support member component. The second support member component may be sized and shaped so that a controller region of a flex circuit can be positioned around it. The intravascular device may omit the second support member component in some instances. Accordingly, the step 1320 may be omitted in such instances.

At step 1330, the method 1300 includes coupling the first and/or second support member components to a flexible, inner proximal member. For example, the proximal member may be threaded through a longitudinal lumen of the first and/or second support member components. Step 1330 can include applying an adhesive to the first support member component, the second support member component, and/or the inner proximal member. The first and/or second support members are spaced from one another when coupled to the inner proximal member.

At step 1340, method 1300 includes positioning the flex circuit around the first and/or second support member components. For example, the flex circuit can be rolled around the first and/or second support member components. A transition region of the flex circuit can extend between the first and second support member components that are spaced from one another. The transducer region of the flex circuit can be in contact with stands of the first support member component that elevate and/or space the flex circuit from a central body portion. In some embodiments, the method 1300 includes introducing an acoustic backing material between the flex circuit and the central body portion of the first support member, such as via passageways of the one or more first support member stand(s). The transition region of the flex circuit can be positioned around the inner support member. The transition region of the flex circuit can be non-rectangular and include one or more cutouts that increase the flexibility of the flex circuit. The controller region of the flex circuit can be positioned in a rolled configuration around the second support member. In embodiments that omit the second support member component, the controller region of the flex circuit can be positioned around the inner proximal member. The flex circuit can be coupled to the first support member component, the second support member component, and/or the inner proximal member via an adhesive.

At step 1350, the method 1300 includes coupling a distal member to the flex circuit and/or the first support member component. For example, the distal member may be slide over a distal flange of the first support member component such that the distal member abuts a distal stand of the first support member component and/or the flex circuit. Adhesive can be applied to the distal member, the distal flange, the distal stand, and/or flex circuit.

At step 1360, the method 1300 includes positioning an outer proximal member around at least a portion of the flex circuit and/or the inner proximal member. For example, the outer proximal member can be slide over the inner proximal member and the controller region and/or the transition region of the flex circuit. Adhesive can coupled the flex circuit, the inner proximal member, and/or the outer proximal member.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An intravascular imaging device comprising:
a flexible elongate member sized and shaped for insertion into a vessel of a patient, the flexible elongate member having a proximal portion and a distal portion;
an imaging assembly disposed at the distal portion of the flexible elongate member, the imaging assembly including:
a flex circuit (914) comprising a controller region (904) at a proximal portion (922) of the flex circuit, a transducer region (908) at a distal portion (920) of the flex circuit, and a transition region (910) extending between the controller region (910) and the transducer region (908);
wherein the flex circuit (914) includes a continuous surface between the controller region (904) and the transducer region (908) and a plurality of conductive traces (216) extending between the controller region (904) and the transducer region (908) within the transition region (910); and wherein the flex circuit (914) comprises a plurality of cutouts (960) extending within the transition region (910); and
a support member (500) around which the flex circuit is positioned, the support member having a proximal portion (502), a distal portion (504), and a central portion (506) having greater flexibility than the proximal and distal portions;
wherein the flex circuit (914) is aligned with the support member (500) such that the transition region (910) of the flex circuit having the conductive traces (216) is aligned with the central portion (506) of the support member (500).

2. The device of claim 1, wherein a value of a dimension of the transition region (910) of the flex circuit (914) is less than values of the corresponding dimension of the transducer region (908) and controller region (904).

3. The device of claim 1, wherein the transition region (910) of the flex circuit (914) is contact with the central portion (506) of the support member (500) when the flex circuit (914) is positioned around the support member (500).

4. The device of claim 1, wherein the value of a dimension of the central portion (506) of the support member (500) is less than a value of the corresponding dimension of the proximal portion (502) and of the distal portion (504).

5. The device of claim 1, wherein the central portion (506) includes a cutout extending radially inwards from an outer surface of the support member (500).

6. The device of claim 1, wherein a shape of the central portion (506) of the support member (500) corresponds to an orientation of at least a portion of the flex circuit (914) around the support member (500).

7. The device of claim 6, wherein the central portion (506) extends i) linearly between the proximal portion (502) and the distal portion (504) or ii) in a curved manner between the proximal portion (502) and the distal portion (504) or iii) in a spiral manner between the proximal portion (502) and the distal portion (504).

8. The device of claim 6, wherein the proximal portion (502), central portion (506), and distal portion (504) of the support member (500) are integrally formed.

9. The device of claim 1, wherein the proximal portion (502) and the distal portion (504) are distinct components mechanically coupled via the central portion (506).

10. The device of claim 1, wherein the central portion (506) of the support member (500) comprises a first material, and the proximal portion (502) and the distal portion (504) comprise a second material different than the first material.

11. A method of assembling an intravascular imaging device, the method comprising:
obtaining (810) a support member having a proximal portion, a distal portion, and a central portion having greater flexibility than the proximal and distal portions;
obtaining a flex circuit (914) comprising a controller region (904) at a proximal portion (922) of the flex circuit, a transducer region (908) at a distal portion (920) of the flex circuit, and a transition region (910) extending between the controller region (910) and the transducer region (908); wherein the flex circuit (914) includes a continuous surface between the controller region (904) and the transducer region (908) and a plurality of conductive traces (216) extending between the controller region (904) and the transducer region (908) within the transition region (910); and wherein the flex circuit (914) comprises a plurality of cutouts (960) extending within the transition region (910)
positioning (820) the flex circuit around the support member, wherein the positioning includes wrapping a portion of the flex circuit around the support member in a spiral configuration such that the flex circuit (914) is aligned with the support member (500) and the transition region (910) of the flex circuit having the conductive traces (216) is aligned with the central portion (506) of the support member (500); and
coupling (830) a flexible elongate member to at least one of the flex circuit or the support member such that the flex circuit and the support member are disposed at a distal portion of the flexible elongate member.

12. The method of claim 11, wherein the obtaining includes:
molding (812) the support member having integrally formed proximal, distal, and central portions.

13. The method of claim 11, wherein the obtaining includes:
removing (814) material from a blank of the support member to form the proximal, distal, and central portions.

14. The method of claim 13, wherein a value of a dimension of the central portion of the support member is less than values of the corresponding dimension of the proximal and distal portions, and wherein the removing forms the central portion.

15. The method of claim 11, wherein the obtaining includes:
obtaining (816) first and second support member components; and
coupling (818) the first and second support member components using a connection member.

## Patentansprüche

1. Intravaskuläre Bildgebungsvorrichtung, umfassend:
ein flexibles längliches Element, das zum Einführen in ein Gefäß eines Patienten bemessen und geformt ist, wobei das flexible längliche Element einen proximalen Abschnitt und einen distalen Abschnitt aufweist;
eine Bildgebungsanordnung, die an dem distalen Abschnitt des flexiblen länglichen Elements angeordnet ist, wobei die Bildgebungsanordnung umfasst:
eine Flex-Schaltung (914), umfassend einen Steuerbereich (904) an einem proximalen Abschnitt (922) der Flex-Schaltung, einen Wandlerbereich (908) an einem distalen Abschnitt (920) der Flex-Schaltung und einem Übergangsbereich (910), der sich zwischen dem Steuerbereich (910) und dem Wandlerbereich (908) erstreckt;
wobei die Flex-Schaltung (914) eine kontinuierliche Oberfläche zwischen dem Steuerbereich (904) und dem Wandlerbereich (908) und mehrere leitende Spuren (216) umfasst, die sich zwischen dem Steuerbereich (904) und dem Wandlerbereich (908) innerhalb des Übergangsbereichs (910) erstrecken; und wobei die Flex-Schaltung (914) mehrere Ausschnitte (960) umfasst, die sich innerhalb des Übergangsbereichs (910) erstrecken; und
ein Stützelement (500), um das die Flex-Schaltung positioniert ist, wobei das Stützelement einen proximalen Abschnitt (502), einen distalen Abschnitt (504) und einen zentralen Abschnitt (506) mit größerer Flexibilität als der proximale und der distale Abschnitt aufweist;
wobei die Flex-Schaltung (914) mit dem Stützelement (500) so ausgerichtet ist, dass der Übergangsbereich (910) der Flex-Schaltung mit den leitenden Spuren (216) mit dem Mittelabschnitt (506) des Stützelements (500) ausgerichtet ist.

2. Vorrichtung nach Anspruch 1, wobei ein Wert einer Abmessung des Übergangsbereichs (910) der Flex-Schaltung (914) kleiner ist als Werte der entsprechenden Abmessung des Wandlerbereichs (908) und des Steuerungsbereichs (904).

3. Vorrichtung nach Anspruch 1, wobei der Übergangsbereich (910) der Flex-Schaltung (914) mit dem Mittelabschnitt (506) des Stützelements (500) in Kontakt steht, wenn die Flex-Schaltung (914) um das Stützelements (500) herum positioniert ist.

4. Vorrichtung nach Anspruch 1, wobei der Wert einer Abmessung des Mittelabschnitts (506) des Stützelements (500) kleiner ist als ein Wert der entsprechenden Abmessung des proximalen Abschnitts (502) und des distalen Abschnitts (504).

5. Vorrichtung nach Anspruch 1, wobei der Mittelabschnitt (506) einen Ausschnitt aufweist, der sich radial nach innen von einer Außenfläche des Stützelements (500) erstreckt.

6. Vorrichtung nach Anspruch 1, wobei eine Form des Mittelabschnitts (506) des Stützelements (500) einer Ausrichtung mindestens eines Abschnitts der Flex-Schaltung (914) um das Stützelement (500) entspricht.

7. Vorrichtung nach Anspruch 6, wobei sich der Mittelabschnitt (506) i) linear zwischen dem proximalen Abschnitt (502) und dem distalen Abschnitt (504) oder ii) in einer gekrümmten Weise zwischen dem proximalen Abschnitt (502) und dem distalen erstreckt Abschnitt (504) oder iii) spiralförmig zwischen dem proximalen Abschnitt (502) und dem distalen Abschnitt (504) erstreckt.

8. Vorrichtung nach Anspruch 6, wobei der proximale Abschnitt (502), der Mittelabschnitt (506) und der distale Abschnitt (504) des Stützelements (500) einstückig ausgebildet sind.

9. Vorrichtung nach Anspruch 1, wobei der proximale Abschnitt (502) und der distale Abschnitt (504) unterschiedliche Komponenten sind, die mechanisch über den Mittelabschnitt (506) gekoppelt sind.

10. Vorrichtung nach Anspruch 1, wobei der Mittelabschnitt (506) des Stützelements (500) ein erstes Material umfasst und der proximale Abschnitt (502) und der distale Abschnitt (504) ein zweites Material umfassen, das sich von dem ersten Material unterscheidet.

11. Verfahren zum Zusammenbau einer intravaskulären Bildgebungsvorrichtung, wobei das Verfahren umfasst:
Erhalten (810) eines Stützelements mit einem proximalen Abschnitt, einem distalen Abschnitt und einem zentralen Abschnitt mit einer größeren Flexibilität als der proximale und der distale Abschnitt;
Erhalten einer Flex-Schaltung (914), umfassend einen Steuerbereich (904) an einem proximalen Abschnitt (922) der Flex-Schaltung, einen Wandlerbereich (908) an einem distalen Abschnitt (920) der Flex-Schaltung und einen Übergangsbereich (910), der sich zwischen dem Steuerbereich (910) und dem Wandlerbereich (908) erstreckt; wobei die Flex-Schaltung (914) eine kontinuierliche Oberfläche zwischen dem Steuerbereich (904) und dem Wandlerbereich (908) und mehrere leitende Spuren (216) umfasst, die sich zwischen dem Steuerbereich (904) und dem Wandlerbereich (908) innerhalb des Übergangsbereichs (910) erstrecken; und wobei die Flex-Schaltung (914) mehrere Ausschnitte (960) umfasst, die sich innerhalb des Übergangsbereichs (910) erstrecken;
Positionieren (820) der Flex-Schaltung um das Stützelement, wobei das Positionieren das Wickeln eines Abschnitts der Flex-Schaltung um das Stützelement in einer Spiralkonfiguration umfasst, so dass die Flex-Schaltung (914) mit dem Stützelement (500) ausgerichtet ist, und der Übergangsbereich (910) der Flex-Schaltung mit den leitenden Spuren (216) mit dem Mittelabschnitt (506) des Stützelements (500) ausgerichtet ist; und
Koppeln (830) eines flexiblen länglichen Elements mit mindestens einer der Flex-Schaltung oder des Stützelements, so dass die Flex-Schaltung und das Stützelement an einem distalen Abschnitt des flexiblen länglichen Elements angeordnet sind.

12. Verfahren nach Anspruch 11, wobei das Erhalten umfasst:
Ausformen (812) des Stützelements mit integral ausgebildeten proximalen, distalen und zentralen Abschnitten.

13. Verfahren nach Anspruch 11, wobei das Erhalten umfasst:
Entfernen (814) von Material von einem Rohling des Stützelements, um den proximalen, distalen und zentralen Abschnitt zu bilden.

14. Verfahren nach Anspruch 13, wobei ein Wert einer Abmessung des Mittelabschnitts des Stützelements kleiner ist als Werte der entsprechenden Abmessung des proximalen und distalen Abschnitts, und wobei das Entfernen den Mittelabschnitt bildet.

15. Verfahren nach Anspruch 11, wobei das Erhalten umfasst:
Erhalten (816) von ersten und zweiten Stützelementkomponenten; und
Koppeln (818) von ersten und zweiten Stützelementkomponenten unter Verwendung eines Verbindungselements.

## Revendications

1. Dispositif d'imagerie intravasculaire comprenant:
un élément allongé flexible dimensionné et façonné pour être inséré dans un vaisseau d'un patient, l'élément allongé flexible ayant une portion proximale et une portion distale;
un ensemble d'imagerie disposé à la portion distale de l'élément allongé flexible, l'ensemble d'imagerie comprenant:
un circuit flex (914) comprenant une région de commande (904) au niveau d'une portion proximale (922) du circuit flex, une région de transducteur (908) au niveau d'une portion distale (920) du circuit flex, et une région de transition (910) s'étendant entre la région de commande (910) et la région de transducteur (908);
où le circuit flex (914) comprend une surface continue entre la région de commande (904) et la région de transducteur (908) et une pluralité de traces conductrices (216) s'étendant entre la région de commande (904) et la région de transducteur (908) à l'intérieur de la région de transition (910); et où le circuit flex (914) comprend une pluralité de entailles (960) s'étendant à l'intérieur de la région de transition (910); et
un élément de support (500) autour duquel le circuit flex est positionné, l'élément de support ayant une portion proximale (502), une portion distale (504) et une portion centrale (506) ayant une plus grande flexibilité que les portions proximale et distale;
où le circuit flex (914) est aligné avec l'élément de support (500) de telle sorte que la région de transition (910) du circuit flex ayant les traces conductrices (216) est alignée avec la portion centrale (506) de l'élément de support (500).

2. Dispositif selon la revendication 1, dans lequel une valeur d'une dimension de la région de transition (910) du circuit flex (914) est inférieure aux valeurs de la dimension correspondante de la région de transducteur (908) et de la région de commande (904) .

3. Dispositif selon la revendication 1, dans lequel la région de transition (910) du circuit flex (914) est en contact avec la portion centrale (506) de l'élément de support (500) lorsque le circuit flex (914) est positionné autour de l'élément de support (500) .

4. Dispositif selon la revendication 1, dans lequel la valeur d'une dimension de la portion centrale (506) de l'élément de support (500) est inférieure à une valeur de la dimension correspondante de la portion proximale (502) et de la portion distale (504) .

5. Dispositif selon la revendication 1, dans lequel la portion centrale (506) comprend une entaille s'étendant radialement vers l'intérieur à partir d'une surface extérieure de l'élément de support (500) .

6. Dispositif selon la revendication 1, dans lequel une forme de la portion centrale (506) de l'élément de support (500) correspond à une orientation d'au moins une portion du circuit flex (914) autour de l'élément de support (500).

7. Dispositif selon la revendication 6, dans lequel la portion centrale (506) s'étend i) linéairement entre la portion proximale (502) et la portion distale (504) ou ii) de manière incurvée entre la portion proximale (502) et la portion distale (504) ou iii) en spirale entre la portion proximale (502) et la portion distale (504).

8. Dispositif selon la revendication 6, dans lequel la portion proximale (502), la portion centrale (506), et la portion distale (504) de l'élément de support (500) sont formées intégralement.

9. Dispositif selon la revendication 1, dans lequel la portion proximale (502) et la portion distale (504) sont des composants distincts couplés mécaniquement via la portion centrale (506).

10. Dispositif selon la revendication 1, dans lequel la portion centrale (506) de l'élément de support (500) comprend un premier matériau, et la portion proximale (502) et la portion distale (504) comprennent un deuxième matériau différent du premier matériau.

11. Procédé d'assemblage d'un dispositif d'imagerie intravasculaire, le procédé comprenant:
l'obtention (810) d'un élément de support ayant une portion proximale, une portion distale et une portion centrale ayant une plus grande flexibilité que les portions proximale et distale;
l'obtention d'un circuit flex (914) comprenant une région de commande (904) au niveau d'une portion proximale (922) du circuit flex, une région de transducteur (908) au niveau d'une portion distale (920) du circuit flex, et une région de transition (910) s'étendant entre la région de commande (910) et la région de transducteur (908); où le circuit flex (914) comprend une surface continue entre la région de commande (904) et la région de transducteur (908) et une pluralité de traces conductrices (216) s'étendant entre la région de commande (904) et la région de transducteur (908) à l'intérieur la région de transition (910); et où le circuit flex (914) comprend une pluralité de entailles (960) s'étendant à l'intérieur de la région de transition (910);
le positionnement (820) du circuit flex autour de l'élément de support, où le positionnement comprend enrouler une portion du circuit flex autour de l'élément de support dans une configuration en spirale de telle sorte que le circuit flex (914) est aligné avec l'élément de support (500) et la région de transition (910) du circuit flex ayant les traces conductrices (216) est alignée avec la portion centrale (506) de l'élément de support (500); et
le couplage (830) d'un élément allongé flexible à au moins l'un du circuit flex ou de l'élément de support de telle sorte que le circuit flex et l'élément de support sont disposés au niveau d'une portion distale de l'élément allongé flexible.

12. Procédé selon la revendication 11, dans lequel l'obtention comprend:
le moulage (812) de l'élément de support ayant des portions proximale, distale et centrale formées intégralement.

13. Procédé selon la revendication 11, dans lequel l'obtention comprend:
l'enlèvement (814) de matériau d'une ébauche de l'élément de support pour former les portions proximale, distale et centrale.

14. Procédé selon la revendication 13, dans lequel une valeur d'une dimension de la portion centrale de l'élément de support est inférieur à des valeurs de la dimension correspondante des portions proximale et distale, et dans lequel l'enlèvement forme la portion centrale.

15. Procédé selon la revendication 11, dans lequel l'obtention comprend:
l'obtention (816) des premier et deuxième composants de l'élément de support; et
le couplage (818) des premier et deuxième composants de l'élément de support à l'aide d'un élément de connexion.
